# EUROPEAN PATENT SPECIFICATION

(11) **EP 1 485 078 B1**
(45) Date of publication and mention of the grant of the patent: **26.09.2012**
(21) Application number: 03744697.8
(22) Date of filing: 17.03.2003
(51) Int. Cl.: A61K 31/165, A61P 25/00

(54) **Milnacipran for the treatment of irritable bowel syndrome**
Milnacipran zur Behandlung von Reizdarmsyndrom
Milnacipran pour le traitement du syndrôme du colon irritable

(30) Priority: 15.03.2002 US 364531 P
(43) Date of publication of application: 15.12.2004
(73) Proprietor: Cypress Bioscience, Inc., San Diego, CA 92121 (US)
(72) Inventor: RAO, Srinivas, G., San Diego, CA 92131 (US); KRANZLER, Jay, D., La Jolla, CA 92037 (US)
(74) Representative: Olsen, Lars Pallisgaard
(86) International application number: PCT/US2003/008155
(87) International publication number: WO 2003/077897

(56) References cited:
- WO-A-00/28980
- WO-A-01/26623
- WO-A-97/33880
- WO-A-02/060428
- US-A- 6 150 396
- EISEN A: "Venlafaxine therapy for vulvodynia." PAIN CLINIC, vol. 8, no. 4, 1996, pages 365-367, XP009014479 ISSN: 0169-1112
- TALLEY N J ET AL: "Irritable bowel syndrome: a little understood organic bowel disease?" LANCET, XX, XX, vol. 360, no. 9332, 17 August 2002 (2002-08-17), pages 555-564, XP004376467 ISSN: 0140-6736
- MORET C ET AL: "BIOCHEMICAL PROFILE OF MIDALCIPRAN (F 2207), 1-PHENYL-U-DIETHYL-AMINOCARBONYL-2-AMINOME THYL-CYCLOPROPANE (Z) HYDROCHLORIDE, A POTENTIAL FOURTH GENERATION ANTIDEPRESSANT DRUG" NEUROPHARMACOLOGY, PERGAMON PRESS, OXFORD, GB, vol. 24, no. 12, 1 December 1985 (1985-12-01), pages 1211-1219, XP000195985 ISSN: 0028-3908
- SHUTO S ET AL: "SYNTHESIS AND BIOLOGICAL ACTIVITY OF CONFORMATIONALLY RESTRICTED ANALOGS OF MILNACIPRAN: (1S,2R)-1-PHENYL-2-U(S)-1-AMINOPROPYL)-N,N -DI ETHYLCYCLOPROPANECARBOXAMIDE, AN EFFICIENT NONCOMPETITIVE N- METHYL-D-ASPARTIC ACID RECEPTOR ANTAGONIST" JOURNAL OF MEDICINAL CHEMISTRY, AMERICAN CHEMICAL SOCIETY. WASHINGTON, US, vol. 39, no. 24, 22 November 1996 (1996-11-22), pages 4844-4852, XP000857417 ISSN: 0022-2623 cited in the application

## Description

### Background of the Invention

A common form of pain syndrome observed in the clinical setting is the visceral pain syndrome. Examples of visceral pain syndromes (VPS) include irritable bowel syndrome (IBS), noncardiac chest pain (NCCP), functional dyspepsia, interstitial cystitis, essential vulvodynia, and urethral syndrome. A common feature of the visceral pain syndromes is pain or discomfort arising from the organs and tissues of the thorax, abdomen, and pelvis.

The pain and discomfort felt in the above syndromes is widely believed to be the result of visceral hypersensitivity. One common form of visceral hypersensitivity is visceral hyperalgesia, i.e., increased sensitivity in visceral organs and/or tissues to painful stimuli. Visceral hyperalgesia has been demonstrated in several VPS, including functional gastrointestinal disorders, like IBS, NCCP, and functional dyspepsia. Visceral hyperalgesia is also believed to contribute to other non-gastrointestinal VPS, including interstitial cystitis, essential vulvodynia, and orchiaglia.

Hyperalgesia is believed to be caused by the "sensitization" of the nervous system. Such sensitization can be a result of changes occurring peripherally (i.e., due to inflammation locally within the skin, muscle, bladder, or in the organs of the gastrointestinal tract), centrally (at the level of the spinal cord, brainstem, thalamus, or cortex), or at both locations. Moreover, acute peripheral sensitization can ultimately lead to a state of chronic central sensitization. The mechanisms underlying central sensitizations are complex and can involve alterations in wide variety of neurotransmitter systems. In particular, alteration in NMDA mediated glutamatergic neurotransmission, or alterations in descending "inhibitory" pain pathways whose effects are mediated by norepinephrine and serotonin can result in a centrally mediated hyperalgesic states.

Although there have been significant breakthroughs in the understanding of the pathophysiology of VPS, the treatment of these syndromes present a particularly challenging task for clinicians. Some of the common medications currently employed to treat VPS include, but are not limited to, analgesics, hypnotics, immune suppressants, antidepressants, various other prescribed medications, and an array of non-prescription medications.

Among all the therapeutic agents, the most widely used agents for VPS are the antidepressants. Antidepressants are widely used due to the belief that these agents have both analgesic and psychotropic properties beneficial to the treatment of VPS. However, the broad array of medications used in VPS patients, including the antidepressants, are either not particularly effective in the treatment of these syndromes or their use is limited due to side effects. Thus, there is a need to develop effective treatments for VPS. The ideal agents would reduce the awareness of visceral pain, produce analgesia over a wide range of pain types, act satisfactorily whether given orally or parenterally, produce minimal or no side effects, and be free from the tendency to produce tolerance and drug dependence.

Compounds that inhibit reuptake of both NE and 5-HT, such as venlafaxine, duloxetine, and certain TCAs may be effective for the treatment of visceral pain syndromes (e.g., irritable bowel syndrome), when administered in combination with neurotransmitter precursors such as phenylalanine, tyrosine and/or tryptophan. See, WO 01/26623 and U.S. Patent No. 6,441,038. These references, however, disclose that a compound that inhibits reuptake of both NE and 5-HT was effective only when administered in combination with a neurotransmitter precursor.

### Summary of the Invention

The present invention provides a pharmaceutical composition comprising milnacipran or a pharmaceutically acceptable salt thereof and a pharmaceutically acceptable carrier for use in the therapeutic treatment of a mammal suffering from irritable bowel syndrome (IBS), wherein the medicament does not comprise a neurotransmitter precursor selected from the group consisting of phenylalanine, tyrosine, tryptophan, or a combination thereof.

### Detailed Description of the Invention

Specific values, ranges, substituents, and embodiments provided below are for illustration purposes only, and do not otherwise the scope of the invention, which is defined by the claims. As used herein, the following terms and expressions have the indicated meanings. It will be appreciated that the compounds useful in the present invention can contain asymmetrically substituted carbon atoms, and can be isolated in optically active or racemic forms. It is well known in the art how to prepare optically active forms, such as by resolution of racemic forms or by synthesis, from optically active starting materials.

All chiral, diastereomeric, racemic forms and all geometric isomeric forms of a structure are intended, unless the specific stereochemistry or isomeric form is specifically indicated. Specifically, for the compound of formula (I), the center bearing both the optionally substituted phenyl ring and the C(=O)NR₁R₂ group can be either (R)- or (S)-; and the center bearing the hydrogen and the CH₂NR₃R₄ group can be either (R) - or (S) -. Likewise, for milnacipran, the center bearing both the phenyl ring and the C(=O)N(CH₂)CH₂ group can be either (R)-or (S)-; and the center bearing the hydrogen and the CH₂NH₂ group can be either (R)- or (S)-.

The processes to prepare or manufacture compounds useful in the present invention are contemplated to be practiced on at least a multigram scale, kilogram scale, multikilogram scale, or industrial scale. Multigram scale, as used herein, is preferably the scale wherein at least one starting material is present in 10 grams or more, more preferably at least 50 grams or more, even more preferably at least 100 grams or more. Multi-kilogram scale, as used herein, is intended to mean the scale wherein more than one kilogram of at least one starting material is used. Industrial scale as used herein is intended to mean a scale which is other than a laboratory scale and which is sufficient to supply product sufficient for either clinical tests or distribution to consumers.

"Irritable bowel syndrome" (IBS) is characterized by abdominal pain, bloating, and disturbed defecation. Various diagnostic criteria have been developed for IBS. See Fass et al., 2001, Arch Intern Med, 161:2081-2088. The Rome II diagnostic criteria includes at least 12 weeks, which need not be consecutive, in the preceding 12 months of abdominal discomfort or pain that has two of the following three features: (i) relieved with defecation; and/or (ii) onset associated with a change in frequency of stool; and/or (iii) onset associated with a change in form (appearance) of stool. See Thompson et al., 2000, In: Drossman et al., eds. Rome II: The functional Gastrointestinal Disorders. McLean, Va: Degnon Associates, 351-432. exclude other conditions such as pelvic inflammatory disease, sexually transmitted disease, or bladder cancer. See Ratner, 2001, World J Urol, 19:157-159.

As used herein, "treating" or "treat" includes (i) preventing a pathologic condition from occurring (e.g. prophylaxis); (ii) inhibiting the pathologic condition or arresting its development; and/or (iii) relieving the pathologic condition)).

As used herein, "pharmaceutically acceptable salts" refer to derivatives of the disclosed compounds wherein the parent compound is modified by making acid or base salts thereof. Examples of pharmaceutically acceptable salts include, but are not limited to, mineral or organic acid salts of basic residues such as amines; alkali or organic salts of acidic residues such as carboxylic acids; and the like. The pharmaceutically acceptable salts include the conventional non-toxic salts or the quaternary ammonium salts of the parent compound formed, for example, from non-toxic inorganic or organic acids. For example, such conventional non-toxic salts include those derived from inorganic acids such as hydrochloric, hydrobromic, sulfuric, sulfamic, phosphoric, nitric and the like; and the salts prepared from organic acids such as acetic, propionic, succinic, glycolic, stearic, lactic, malic, tartaric, citric, ascorbic, pamoic, maleic, hydroxymaleic, phenylacetic, glutamic, benzoic, salicylic, sulfanilic, 2-acetoxybenzoic, fumaric, toluenesulfonic, methanesulfonic, ethane disulfonic, oxalic, isethionic, and the like.

The pharmaceutically acceptable salts of the compounds useful in the present invention can be synthesized from the parent compound, which contains a basic or acidic moiety, by conventional chemical methods. Generally, such salts can be prepared by reacting the free acid or base forms of these compounds with a stoichiometric amount of the appropriate base or acid in water or in an organic solvent, or in a mixture of the two; generally, nonaqueous media like ether, ethyl acetate, ethanol, isopropanol, or acetonitrile are preferred. Lists of suitable salts are found in Remington's Pharmaceutical Sciences, 17th ed., Mack Publishing Company, Easton, PA, 1985, p. 1418, the disclosure of which is hereby incorporated by reference.

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication commensurate with a reasonable benefit/risk ratio.

"Stable compound" and "stable structure" are meant to indicate a compound that is sufficiently robust to survive isolation to a useful degree of purity from a reaction mixture, and formulation into an efficacious therapeutic agent. Only stable compounds are contemplated by the present invention.

"Substituted" is intended to indicate that one or more hydrogens on the atom indicated in the expression using "substituted" is replaced with a selection from the indicated group(s), provided that the indicated atom's normal valency is not exceeded, and that the substitution results in a stable compound. Suitable indicated groups include, e.g., alkyl, alkoxy, halo, haloalkyl, hydroxy, hydroxyalkyl, aryl, heteroaryl, heterocycle, cycloalkyl, alkanoyl, alkoxycarbonyl, amino, alkylamino, acylamino, nitro, trifluoromethyl, trifluoromethoxy, carboxy, carboxyalkyl, keto, thioxo, alkylthio, alkylsulfinyl, alkylsulfonyl and cyano. When a substituent is keto (i.e., =O) or thioxo (i.e., =S) group, then 2 hydrogens on the atom are replaced.

"Therapeutically effective amount" is intended to include an amount of a compound useful in the present invention or an amount of the combination of compounds claimed, e.g., to treat visceral pain syndromes. The combination of compounds is preferably a synergistic combination. Synergy, as described for example by Chou and Talalay, Adv. Enzyme Regul. 22:27-55 (1984), occurs when the effect (in this case, treatment of irritable bowel syndrome ) of the compounds when administered in combination is greater than the additive effect of the compounds when administered alone as a single agent. In general, a synergistic effect is most clearly demonstrated at suboptimal concentrations of the compounds. Synergy can be in terms of lower cytotoxicity, increased activity, or some other beneficial effect of the combination compared with the individual components.

"Mammal" refers to an animal of the class Mammalia, and includes humans.

"Prodrugs" are intended to include any covalently bonded substances which release the active parent drug or other formulas or compounds of the present invention in vivo when such prodrug is administered to a mammalian subject. Prodrugs of a compound useful in the present invention, for example milnacipran, are prepared by modifying functional groups present in the compound in such a way that the modifications are cleaved, either in routine manipulation in vivo, to the parent compound. Prodrugs include compounds useful in the present invention wherein the hydroxy or amino group is bonded to any group that, when the prodrug is administered to a mammalian subject, cleaves to form a free hydroxyl or free amino, respectively. Examples of prodrugs include, but are not limited to, acetate, formate and benzoate derivatives of alcohol and amine functional groups in the compounds useful in the present invention, and the like.

"Metabolite" refers to any substance resulting from biochemical processes by which living cells interact with the active parent drug or other formulas or compounds useful in the present invention in vivo, when such active parent drug or other formulas or compounds useful in the present invention are administered to a mammalian subject. Metabolites include products or intermediates from any metabolic pathway.

"Metabolic pathway" refers to a sequence of enzyme-mediated reactions that transform one compound to another and provides intermediates and energy for cellular functions. The metabolic pathway can be linear or cyclic. A specific metabolic pathway includes the glucuronide conjugation.

The term "alkyl" refers to a monoradical branched or unbranched saturated hydrocarbon chain preferably having from 1 to 40 carbon atoms, more preferably 1 to 10 carbon atoms, and even more preferably 1 to 6 carbon atoms. This term is exemplified by groups such as methyl, ethyl, *n-*propyl, *iso*-propyl, *n*-butyl, *iso*-butyl, *sec*-butyl, *n*-hexyl, *n*-decyl, tetradecyl, and the like.

The alkyl can optionally be substituted with one or more alkoxy, halo, haloalkyl, hydroxy, hydroxyalkyl, aryl, heteroaryl, heterocycle, cycloalkyl, alkanoyl, alkoxycarbonyl, amino, alkylamino, acylamino, nitro, trifluoromethyl, trifluoromethoxy, carboxy, carboxyalkyl, keto, thioxo, alkylthio, alkylsulfinyl, alkylsulfonyl and cyano.

The term "alkylene" refers to a diradical branched or unbranched saturated hydrocarbon chain preferably having from 1 to 40 carbon atoms, more preferably 1 to 10 carbon atoms, and even more preferably 1 to 6 carbon atoms. This term is exemplified by groups such as methylene, ethylene, n-propylene, iso-propylene, n-butylene, iso-butylene, sec-butylene, n-hexylene, n-decylene, tetradecylene, and the like.

The alkylene can optionally be substituted with one or more alkoxy, halo, haloalkyl, hydroxy, hydroxyalkyl, aryl, heteroaryl, heterocycle, cycloalkyl, alkanoyl, alkoxycarbonyl, amino, alkylamino, acylamino, nitro, trifluoromethyl, trifluoromethoxy, carboxy, carboxyalkyl, keto, thioxo, alkylthio, alkylsulfinyl, alkylsulfonyl and cyano.

The term "alkoxy" refers to the groups alkyl-O-, where alkyl is defined herein. Preferred alkoxy groups include, e.g., methoxy, ethoxy, n-propoxy, iso-propoxy, n-butoxy, tert-butoxy, sec-butoxy, n-pentoxy, n-hexoxy, 1,2-dimethylbutoxy, and the like.

The alkyoxy can optionally be substituted with one or more alkyl, halo, haloalkyl, hydroxy, hydroxyalkyl, aryl, heteroaryl, heterocycle, cycloalkyl, alkanoyl, alkoxycarbonyl, amino, alkylamino, acylamino, nitro, trifluoromethyl, trifluoromethoxy, carboxy, carboxyalkyl, keto, thioxo, alkylthio, alkylsulfinyl, alkylsulfonyl and cyano.

The term "aryl" refers to an unsaturated aromatic carbocyclic group of from 6 to 20 carbon atoms having a single ring (e.g., phenyl) or multiple condensed (fused) rings, wherein at least one ring is aromatic (e.g., naphthyl, dihydrophenanthrenyl, fluorenyl, or anthryl). Preferred aryls include phenyl, naphthyl and the like.

The aryl can optionally be substituted with one or more alkyl, alkoxy, halo, haloalkyl, hydroxy, hydroxyalkyl, heteroaryl, heterocycle, cycloalkyl, alkanoyl, alkoxycarbonyl, amino, alkylamino, acylamino, nitro, trifluoromethyl, trifluoromethoxy, carboxy, carboxyalkyl, keto, thioxo, alkylthio, alkylsulfinyl, alkylsulfonyl and cyano.

The term "cycloalkyl" refers to cyclic alkyl groups of from 3 to 20 carbon atoms having a single cyclic ring or multiple condensed rings. Such cycloalkyl groups include, by way of example, single ring structures such as cyclopropyl, cyclobutyl, cyclopentyl, cyclooctyl, and the like, or multiple ring structures such as adamantanyl, and the like.

The cycloalkyl can optionally be substituted with one or more alkyl, alkoxy, halo, haloalkyl, hydroxy, hydroxyalkyl, aryl, heteroaryl, heterocycle, alkanoyl, alkoxycarbonyl, amino, alkylamino, acylamino, nitro, trifluoromethyl, trifluoromethoxy, carboxy, carboxyalkyl, keto, thioxo, alkylthio, alkylsulfinyl, alkylsulfonyl and cyano.

The term "halo" refers to fluoro, chloro, bromo, and iodo. Similarly, the term "halogen" refers to fluorine, chlorine, bromine, and iodine.

"Haloalkyl" refers to alkyl as defined herein substituted by 1-4 halo groups as defined herein, which may be the same or different. Representative haloalkyl groups include, by way of example, trifluoromethyl, 3-fluorododecyl, 12,12,12-trifluorododecyl, 2-bromooctyl, 3-bromo-6-chloroheptyl, and the like.

The term "heteroaryl" is defined herein as a monocyclic, bicyclic, or tricyclic ring system containing one, two, or three aromatic rings and containing at least one nitrogen, oxygen, or sulfur atom in an aromatic ring, and which can be unsubstituted or substituted, for example, with one or more, and in particular one to three, substituents, like halo, alkyl, hydroxy, hydroxyalkyl, alkoxy, alkoxyalkyl, haloalkyl, nitro, amino, alkylamino, acylamino, alkylthio, alkylsulfinyl, and alkylsulfonyl. Examples of heteroaryl groups include, but are not limited to, 2H-pyrrolyl, 3H-indolyl, 4H-quinolizinyl, 4nH-carbazolyl, acridinyl, benzo[b]thienyl, benzothiazolyl, β-carbolinyl, carbazolyl, chromenyl, cinnaolinyl, dibenzo[b,d]furanyl, furazanyl, furyl, imidazolyl, imidizolyl, indazolyl, indolisinyl, indolyl, isobenzofuranyl, isoindolyl, isoquinolyl, isothiazolyl, isoxazolyl, naphthyridinyl, naptho[2,3-b], oxazolyl, perimidinyl, phenanthridinyl, phenanthrolinyl, phenarsazinyl, phenazinyl, phenothiazinyl, phenoxathiinyl, phenoxazinyl, phthalazinyl, pteridinyl, purinyl, pyranyl, pyrazinyl, pyrazolyl, pyridazinyl, pyridyl, pyrimidinyl, pyrimidinyl, pyrrolyl, quinazolinyl, quinolyl, quinoxalinyl, thiadiazolyl, thianthrenyl, thiazolyl, thienyl, triazolyl, and xanthenyl. In one embodiment the term "heteroaryl" denotes a monocyclic aromatic ring containing five or six ring atoms containing carbon and 1, 2, 3, or 4 heteroatoms independently selected from the group non-peroxide oxygen, sulfur, and N(Z) wherein Z is absent or is H, O, alkyl, phenyl or benzyl. In another embodiment heteroaryl denotes an ortho-fused bicyclic heterocycle of about eight to ten ring atoms derived therefrom, particularly a benz-derivative or one derived by fusing a propylene, or tetramethylene diradical thereto.

The heteroaryl can optionally be substituted with one or more alkyl, alkoxy, halo, haloalkyl, hydroxy, hydroxyalkyl, aryl, heterocycle, cycloalkyl, alkanoyl, alkoxycarbonyl, amino, alkylamino, acylamino, nitro, trifluoromethyl, trifluoromethoxy, carboxy, carboxyalkyl, keto, thioxo, alkylthio, alkylsulfinyl, alkylsulfonyl and cyano.

The term "heterocycle" refers to a saturated or partially unsaturated ring system, containing at least one heteroatom selected from the group oxygen, nitrogen, and sulfur, and optionally substituted with alkyl or C(=O)OR^{b}, wherein R^{b} is hydrogen or alkyl. Typically heterocycle is a monocyclic, bicyclic, or tricyclic group containing one or more heteroatoms selected from the group oxygen, nitrogen, and sulfur. A heterocycle group also can contain an oxo group (=O) attached to the ring. Non-limiting examples of heterocycle groups include 1,3-dihydrobenzofuran, 1,3-dioxolane, 1,4-dioxane, 1,4-dithiane, 2H-pyran, 2-pyrazoline, 4H-pyran, chromanyl, imidazolidinyl, imidazolinyl, indolinyl, isochromanyl, isoindolinyl, morpholine, piperazinyl, piperidine, piperidyl, pyrazolidine, pyrazolidinyl, pyrazolinyl, pyrrolidine, pyrroline, quinuclidine, and thiomorpholine.

The heterocycle can optionally be substituted with one or more alkyl, alkoxy, halo, haloalkyl, hydroxy, hydroxyalkyl, aryl, heteroaryl, cycloalkyl, alkanoyl, alkoxycarbonyl, amino, alkylamino, acylamino, nitro, trifluoromethyl, trifluoromethoxy, carboxy, carboxyalkyl, keto, thioxo, alkylthio, alkylsulfinyl, alkylsulfonyl and cyano.

Examples of nitrogen heterocycles and heteroaryls include, but are not limited to, pyrrole, imidazole, pyrazole, pyridine, pyrazine, pyrimidine, pyridazine, indolizine, isoindole, indole, indazole, purine, quinolizine, isoquinoline, quinoline, phthalazine, naphthylpyridine, quinoxaline, quinazoline, cinnoline, pteridine, carbazole, carboline, phenanthridine, acridine, phenanthroline, isothiazole, phenazine, isoxazole, phenoxazine, phenothiazine, imidazolidine, imidazoline, piperidine, piperazine, indoline, morpholino, piperidinyl, tetrahydrofuranyl, and the like as well as N-alkoxy-nitrogen containing heterocycles.

Another class of heterocyclics is known as "crown compounds" which refers to a specific class of heterocyclic compounds having one or more repeating units of the formula [-(CH₂-)ₐA-] where a is equal to or greater than 2, and A at each separate occurrence can be O, N, S or P. Examples of crown compounds include, by way of example only, (-(CH₂)₃-NH-]₃, [-((CH₂)₂-O)₄-((CH₂)₂-NH)₂] and the like. Typically such crown compounds can have from 4 to 10 heteroatoms and 8 to 40 carbon atoms.

The term "alkanoyl" refers to C(=O)R, wherein R is an alkyl group as previously defined.

The term "alkoxycarbonyl" refers to C(=O)OR, wherein R is an alkyl group as previously defined.

The term "amino" refers to -NH₂, and the term "alkylamino" refers to -NR₂, wherein at least one R is alkyl and the second R is alkyl or hydrogen. The term "acylamino" refers to RC(=O)N, wherein R is alkyl or aryl.

The term "nitro" refers to -NO₂.

The term "trifluoromethyl" refers to -CF₃.

The term "trifluoromethoxy" refers to -OCF₃.

The term "cyano" refers to -CN.

The term "hydroxy" refers to -OH.

As to any of the above groups, which contain one or more substituents, it is understood, of course, that such groups do not contain any substitution or substitution patterns which are sterically impractical and/or synthetically non-feasible. In addition, the compounds of this invention include all stereochemical isomers arising from the substitution of these compounds.

One diastereomer of a compound disclosed herein may display superior activity compared with the other. When required, separation of the racemic material can be achieved by HPLC using a chiral column or by a resolution using a resolving agent such as camphonic chloride as in Thomas J . Tucker, et al., J. Med. Chem. 1994 37, 2437-2444. A chiral compound useful in the present invention may also be directly synthesized using a chiral catalyst or a chiral ligand, e.g. Mark A. Huffman, et al., J. Org. Chem. 1995, 60, 1590-1594.

### Selective serotonin (5-HT) norepiniphrine (NE) reuptake inhibitors (SNRI)

The terms "serotonin (5-HT) reuptake" and "norepiniphrine (NE) reuptake" refer to the uptake of the 5-HT or NE from the synaptic cleft by a presynaptic neuron after release of the neurotransmitter by the same neuron in synaptic transmission. The original release of the neurotransmitter into the synaptic cleft by the presynaptic neuron triggers an action potential in the postsynaptic neuron. Reuptake of the neurotransmitter allows the resting potential of the postsynaptic neuron to be restored, clearing the way for it to receive another transmission.

Compounds that can act as selective norepinephrine (NE) - serotonin (5-HT) reuptake inhibitors (NSRIs) include compounds of formula (Ia) : 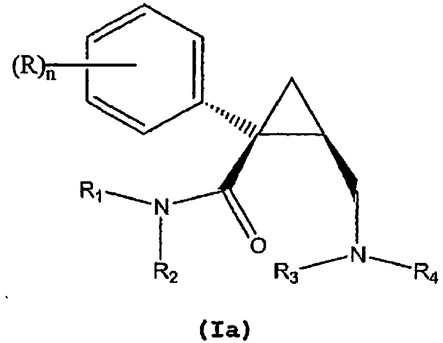 or sterioisomeric forms, mixtures of sterioisomeric forms, or pharmaceutically acceptable salts thereof wherein,
R is independently hydrogen, halo, alkyl, substituted alkyl, alkoxy, substituted alkoxy, hydroxy, nitro, amino, or substituted amino;
n is 1 or 2;
R₁ and R₂ are each independently hydrogen, alkyl, substituted alkyl, aryl, substituted aryl, cycloalkyl, substituted cycloalkyl, alkaryl, substituted alkaryl, heteroaryl, substituted heteroaryl, heterocycle, or substituted heterocycle; or
R₁ and R₂ can form a heterocycle, substituted heterocycle, heteroaryl, or substituted heteroaryl with the adjacent nitrogen atom;
R₃ and R₄ are each independently hydrogen, alkyl, or substituted alkyl; or
R₃ and R₄ can form a heterocycle, substituted heterocycle, heteroaryl, or substituted heteroaryl with the adjacent nitrogen atom.

Additional compounds that can act as selective norepinephrine (NE) - serotonin (5-HT) reuptake inhibitors (NSRIs) include compounds of formula (V): 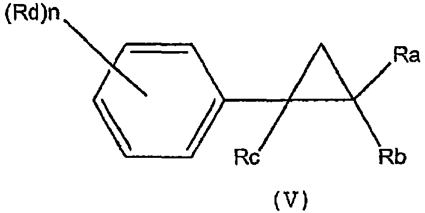 wherein,
Rₐ is hydrogen, alkyl, substituted alkyl, COORₑ or NRₑRₑ; wherein each Rₑ is independently hydrogen, alkyl, or substituted alklyl;
R_{b} is hydrogen, alkyl, substituted alkyl, COORₑ or NRₑRₑ; wherein each Rₑ is independently hydrogen, alkyl, or substituted alkyl; or R_{b} together with R_{c} forms an alkylene chain or a substituted alklylene chain;
R_{c} is hydrogen, alkyl, substituted alkyl, COORₑ or NRₑRₑ; wherein each Rₑ is independently hydrogen, alkyl, or substituted alklyl; or R_{c} together with R_{b} forms an alkylene chain or a substituted alklylene chain;
R_{d} is hydrogen, halo, hydroxy, alkoxy, nitro, COORₐ or NRₑRₑ; wherein each Rₑ is independently hydrogen, alkyl, or substituted alklyl;
n is 1, 2, 3, 4, or 5;
or sterioisomeric forms, mixtures of sterioisomeric forms, or pharmaceutically acceptable salts thereof.

Additional compounds that can act as selective norepinephrine (NE) - serotonin (5-HT) reuptake inhibitors (NSRIs) include compounds of formula (VI)-(XV): 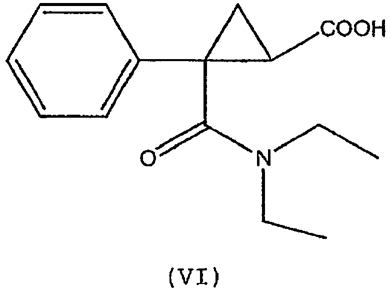 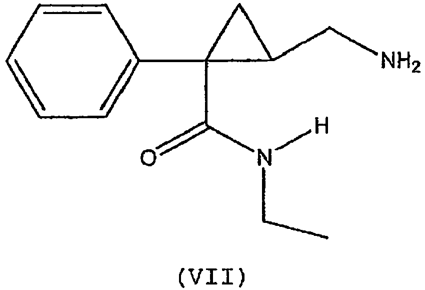 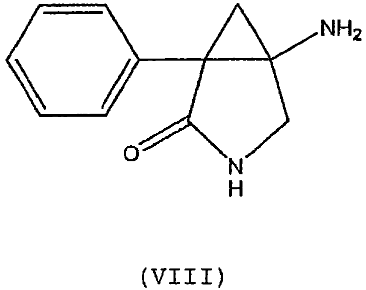 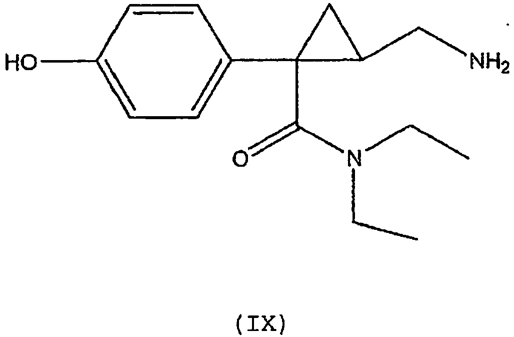 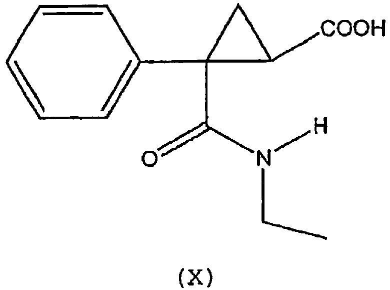 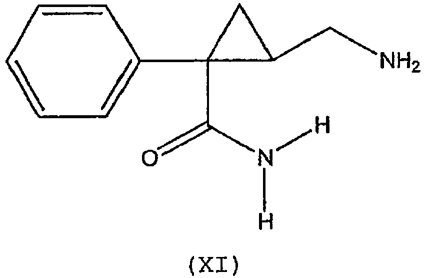 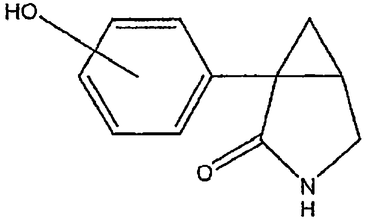 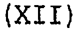 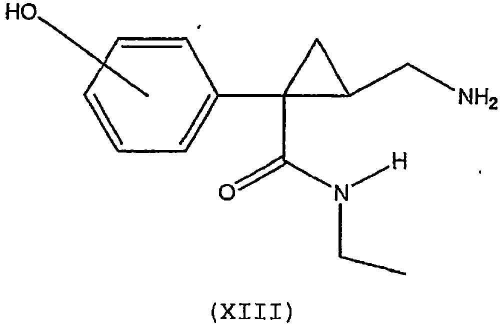 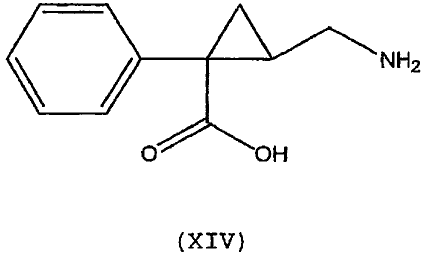 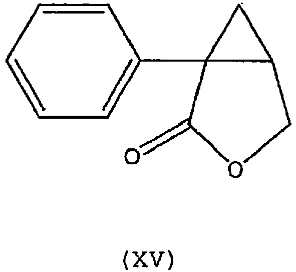 or sterioisomeric forms, mixtures of sterioisomeric forms, or pharmaceutically acceptable salts thereof.

The term "selective serotonin (5-HT) reuptake inhibitor" refers to a compound that has an IC₅₀ for sodium-dependent 5-HT reuptake into rat cerebral cortical synaptosomes of 200 nM or less, and an IC₅₀ for sodium-dependent dopamine uptake into rat striatum synaptosomes of at least 1000 nM, as assayed in Mochizuki, D., et al., Psychopharmacology 162:323-332 (2002). Assays for 5-HT reuptake inhibition activity can also be conducted with recombinant human 5-HT transporter expressed in a cell line in vitro, such as the LLC-PK1 cell line, as reported in Gu et al. J. Biol. Chem. 269:7124-7130 (1994).

Specifically, the IC₅₀ for 5-HT reuptake is 100 nM or less, and for dopamine reuptake is 5 µM or more.

The term "selective norepinephrine (NE) reuptake inhibitor" refers to a compound that has an IC₅₀ for sodium-dependent NE reuptake into rat cerebral cortical synaptosomes of 200 nM or less, and an IC₅₀ for sodium-dependent dopamine uptake into rat striatum synaptosomes of at least 1000 nM, as assayed in Mochizuki, D., et al., Psychopharmacology 162:323-332 (2002). Specifically the IC₅₀ for NE reuptake is 100 nM or less, and for dopamine reuptake is 5 µM or more.

In particular, the selective NE reuptake inhibitor also has an IC₅₀ for sodium-dependent 5-HT reuptake of 300 nM or greater, or of 1000 nM or greater.

The term "selective norepinephrine (NE) - serotonin (5-HT) reuptake inhibitor (NSRI)" refers to a compound that is both a selective NE reuptake inhibitor and a selective 5-HT reuptake inhibitor. Specifically, an NSRI has an IC₅₀ for 5-HT reuptake of 200 nM or less and an IC50 for NE reuptake of 200 nM or less, and an IC₅₀ for dopamine reuptake of at least 1000 nM. The NSRI will have an NE:5-HT reuptake inhibition ratio of at least about 1:1. The NE:5-HT reuptake inhibition ratio is calculated by dividing the IC₅₀ for 5-HT reuptake by the IC₅₀ for NE reuptake. For instance, if a compound has an IC₅₀ for NE reuptake of 10 nM and an IC₅₀ for 5-HT reuptake of 20 nM, it has an NE:5-HT reuptake inhibition ratio of 2:1. In Specifically, the NSRI will have an NE:5-HT reuptake inhibition ratio of about 1:1 to about 20:1, about 1.1:1 to about 20:1, about 1:1 to 5:1, about 1.1:1 to about 5:1, about 1:1 to about 3:1, or about 1.1:1 to about 3:1.

As used herein, selective NSRIs do not include tricyclic antidepressants (TCAs).

Specifically, the NSRI has an IC50 for sodium-dependent dopamine reuptake of at least 5 µM.

Additional norepinephrine (NE) - serotonin (5-HT) reuptake inhibitors (NSRIs) include, e.g., aminocyclopropane derivatives, sibutramine, venlafaxine, and duloxetine.

"Sibutramine" refers to cyclobutanemethaneamine or 1(4-chlorophenyl)-N,N-dimethyl-α-(2-methylpropyl)-, hydrochloride monohydrate. The CAS Registry Numbers are 125494-59-9 [monohydrate], 84485-00-7 [anhydrous]; and 106650-56-0 [sibutramine].

The term "aminocyclopropane derivative" refers to any aminocyclopropane compound possessing suitable selective norepinephrine (NE) - serotonin (5-HT) reuptake inhibition. Suitable aminocyclopropane derivatives are disclosed, e.g., in U.S. Patent No. 5,621,142; WO95/22521; Shuto et al., J. Med. Chem., 38:2964-2968, 1995; Shuto et al., J. Med. Chem., 39:4844-4852, 1996; Shuto et al., J. Med. Chem., 41:3507-3514, 1998; and Shuto et al., J. Med. Chem., 85:207-213, 2001; and Jpn. J. Pharmacol. 85:207-213.

"Venlafaxine" refers to (±)-1-[α-[dimethylamino)methyl]-p-methoxybenzyl]cyclohexanol hydrochloride. The CAS registry Numbers are 99300-78-4; 93413-69-5. Venlafaxine and synthetic preparations for the same are disclosed, e.g., in U.S. Patent Nos. 4,535,186; 4,761,501; and references cited therein. Venlafaxine and methods for its synthesis are described in U.S. Patent 4,535,186, and U.S. Patent 4,761,501. Additional information regarding venlafaxine may be found in the Merck Index, 12th Edition, at entry 10079. It is understood that "venlafaxine" refers to venlafaxine's free base, its pharmaceutically acceptable salts, its racemate and its individual enantiomers, and venlafaxine analogs, both as racemates and as their individual enantiomers. It has been reported that the main metabolite of venlafaxine is *O-*demethylvenlafaxine. See Sanchez et al., 1999, Cellular and Molecular Neurobiology 19(4):467-489. Accordingly, the use of O-demethylvenlafaxine is also within the scope of this invention.

"Duloxetine" refers to 2-thiophenepropanamine, N-methyl-γ-(1-naphthalenyloxy)-hydrochloride. The CAS Registry Number is 116539-59-4. Duloxetine and synthetic preparations for the same are disclosed, e.g., in U.S. Patent No. 4,956,388; and references cited therein. Duloxetine is typically administered to humans as the hydrochloride salt. Duloxetine and methods for its synthesis are described in U.S. Patent 4,956,388. Additional information regarding milnacipran may be found in the Merck Index, 12th Edition, at entry 3518.

### Milnacipran (MIL)

"Milnacipran" or "MIL" refers to (±)-*cis*-2-(aminomethyl)-*N,N*-diethyl-1-phenylcyclopropanecarboxamide. The CAS Registry Number is 92623-85-3. Methods of preparing milnacipran are disclosed, e.g., in U.S. Patent No. 4,478,836 and references cited therein. In humans, milnacipran and its para-hydroxylated derivative are found in urine (Caccia, 1998, Clin Pharmacokinet 34(4):281-302). Accordingly, the para-hydroxylated derivative of milnacipran is particularly useful in the practice of the present invention.

It is believed that that the dextrogyral enantiomer of milnacipran is about twice as active in inhibiting norepinephrine and serotonin reuptake than the racemic mixture, and that the levrogyral enantiomer is much less potent. See, e.g., Viazzo et al., 1996, Tetrahedron Lett. 37(26):4519-4522; Deprez et al., 1998, Eur. J. Drug Metab. Pharmacokinet. 23(2): 166-171). Accordingly, milnacipran can be administered in enantiomerically pure form (e.g., the pure dextrogyral enantiomer) or as a mixture of dextrogyral and levrogyral enantiomers, such as a racemic mixture.

The NE:5-HT of milnacipran is about 2:1. See, Moret, C., M. Charveron, et al. (1985). "Biochemical profile of midalcipran (F 2207), 1-phenyl-1-diethyl- aminocarbonyl-2-aminomethyl-cyclopropane (Z) hydrochloride, a potential fourth generation antidepressant drug." Neuropharmacology 24(12): 1211-9.) Palmier, C., C. Puozzo, et al. (1989). "Monoamine uptake inhibition by plasma from healthy volunteers after single oral doses of the antidepressant milnacipran." Eur J Clin Pharmacol 37(3): 235-8. Milnacipran and synthetic preparations of the same are described in U.S. Patent 4,478,836, and references cited therein. Additional information regarding milnacipran may be found in the Merck Index, 12th Edition, at entry 6281.

Milnacipran is typically administered to adults at a dose of 50 mg BID (taken with meals). Milnacipran can be administered to children at lower doses, e.g., up to about 40 mg BID (taken with meals), up to about 30 mg BID (taken with meals), up to about 20 mg BID (taken with meals), or up to about 10 mg BID (taken with meals).

Additionally, while milnacipran is typically administered to adults at a dose of about 100 mg/70 kg body weight, it can be administered to children at a dose of up to about 60 mg/50 kg body weight, up to about 50 mg/50 kg body weight, up to about 30 mg/50 kg body weight, or up to about 20 mg/50 kg body weight. Specifically, milnacipran can be administered to children at a dose of about 1 mg/50 kg body weight to about 60 mg/50 kg body weight, about 5 mg/50 kg body weight to about 50 mg/50 kg body weight, about 5 mg/50 kg body weight to about 30 mg/50 kg body weight, or about 5 mg/50 kg body weight to about 20 mg/50 kg body weight.

### Combination therapy

Milnacipran can be administered adjunctively with other active compounds such as a medicament for the treatment of dysphagia, dyspepsia, aerophagia, irritable bowel syndrome, abdominal bloating, constipation, diarrhea, abdominal pain, abdominal migraine, gallbladder dysfunction, sphincter of Oddi dysfunction, fecal incontinence, anorectal pain, proctalgia fugax, dyssynergia, dyschezia, vulvodynia, orchialgia, urethral syndrome, penile pain, prostatodynia, coccygodynia, perineal pain, rectal pain, or a combination thereof.

The anorectal pain can include ischemia, inflammatory bowel disease, cryptitis, intramuscular abscess, fissure, hemorrhoids, prostatitis, solitary rectal ulcer, or a combination thereof.

The vulvodynia can include vulvar dermatoses, cyclic vulvovaginitis, vulvar vestibulitis, vulvar papillomatosis, dysesthetic vulvodynia, or a combination thereof.

Specifically, milnacipran can be administered adjunctively with an antidepressant, an antidiarrheal, an analgesic, an antispasmodic, an antifatigue agent, an anorectic, a stimulant, an antiepileptic drug, a sedative/hypnotic, a laxative, a 5-HT₁ agonist, an alpha adrenergic agonist, or a combination thereof.

More specifically, milnacipran can be administered adjunctively with a serotonin reuptake inhibitor, a heterocyclic antidepressant, a monoamine oxidase inhibitor, serotonergicnoradrenergic, a 5-HT₂ antagonist, catecholaminergic, an anticholinergic, a 5-HT₃ receptor antagonist, paregoric, glucose-electrolyte solution, an opiate, an opioid agonist, a NSAID, an indole, a naphthylalkanone, oxicam, a para-aminophenol derivative, propionic acid, salicylate, fenamate, a pyrazole, a salicylate, a gut analgesic, a belladonna alkaloid, nitroglycerin, an anticholinergic, a calcium channel blocker, a corticosteroid, a glucocorticoid, acetazolamide, carbamazepine, clonazepam, ethosuximide, fosphenytoin, gabapentin, lamotrigine, phenobarbital, phenytoin, primidone, topiramate, valproate, a barbiturate, benzodiazepine, imidazopyridine, nondepolarizing neuromuscular blocking agent, a stool softener, a bulk forming agent, alosetron, amphetamine, atropine, buprenorphine, buspirone, carbamazepine, clonidine, codeine, dicyclomine, 1-DOPA, hyoscyamine, lactose, lidocaine, loperamide, mineral oil, modafinil, morphine, neurotonin, octreotide, opiates, phenolpthyaline, pramipexole, pregabalin, psyllium, sibutramine, tegaserod, tizanidine, tramadol, trazodone, tropisetron, valium, zolpidem, .zopiclone, or a combination thereof.

### Specific embodiments:

1. A pharmaceutical composition comprising milnacipran or a pharmaceutically acceptable salt thereof and a pharmaceutically acceptable carrier for use in the therapeutic treatment of a mammal suffering from irritable bowel syndrome (IBS), wherein the pharmaceutical composition does not comprise a neurotransmitter precursor selected from the group consisting of phenylalanine, tyrosine, tryptophan, or a combination thereof.
2. The pharmaceutical composition for use of item 1, further comprising an antidepressant, an anti-diarrheal, an analgesic, an antispasmodic, an anti-fatigue agent, an anorectic, an antiepileptic drug, a sedative/hypnotic, a laxative, or a combination thereof.
3. The pharmaceutical composition for use of item 1, further comprising pramipexole.
4. The pharmaceutical composition for use of item 1, further comprising pregabalin.
5. The pharmaceutical composition for use of item 1, further comprising neurotonin.
6. The pharmaceutical composition for use of item 1 wherein the milnacipran is administered up to about 400 mg/day.
7. The pharmaceutical composition for use of item 1 wherein the milnacipran is administered in about 25 mg/day to about 250 mg/day.

### Utility

The compounds disclosed herein (i.e., those useful in the present invention) possess suitable anti-visceral pain syndrome activity and are therefore useful as agents for the treatment of irritable bowel syndrome.

The compounds disclosed herein are also useful as standard or reference compounds for use in tests or assays for determining the ability of an agent to treat, prevent, or lessen the conditions or symptoms associated with irritable bowel syndrome for example in a pharmaceutical research program. Thus, the compounds disclosed herein may be used as control or reference compound in such assays and as a quality control standard. The compounds of the present invention may be provided in a commercial kit or container for use as such standard or reference compound.

As used herein, "µg" denotes microgram, "mg" denotes milligram, "g" denotes gram, "µL" denotes microliter, "mL" denotes milliliter, "L" denotes liter, "nM" denotes nanomolar, "µM" denotes micromolar, "mM" denotes millimolar, "M" denotes molar and "nm" denotes nanometer. "Sigma" stands for the Sigma-Aldrich Corp. of St. Louis MO.

### Dosage and Formulation

The compounds can be administered as treatment for irritable bowel syndrome by any means that produces contact of the active agent with the agent's site of action in the body of a mammal. They can be administered by any conventional means available for use in conjunction with pharmaceuticals, either as individual therapeutic agents or in a combination of therapeutic agents. They can be administered alone, but preferably are administered with a pharmaceutical carrier selected on the basis of the chosen route of administration and standard pharmaceutical practice.

The dosage administered will, of course, vary depending upon known factors, such as the pharmacodynamic characteristics of the particular agent and its mode and route of administration; the age, health and weight of the recipient; the nature and extent of the symptoms; the kind of concurrent treatment; the frequency of treatment; and the effect desired. A daily dosage of active ingredient can be expected to be about 0.001 to about 1000 milligrams per kilogram of body weight, with the preferred dose being about 0.1 to about 100 mg/kg, preferably administered several times a day.

Dosage forms of compositions suitable for administration contain from about 20 mg to about 500 mg of active ingredient per unit. In these pharmaceutical compositions the active ingredient will ordinarily be present in an amount of about 0.5-95% by weight based on the total weight of the composition. The active ingredient can be administered orally in solid dosage forms, such as capsules, tablets and powders, or in liquid dosage forms, such as elixirs, syrups and suspensions. It can also be administered parenterally, in sterile liquid dosage forms. Additives may also be included in the formulation to enhance the physical appearance, improve stability, and aid in disintegration after administration. For example, liquid dosage forms for oral administration can contain coloring and flavoring to increase patient acceptance.

Gelatin capsules contain the active ingredient and powdered carriers, such as lactose, starch, cellulose derivatives, magnesium stearate, stearic acid, and the like. Similar diluents can be used to make compressed tablets. Both tablets and capsules can be manufactured as sustained release products to provide for continuous release of medication over a period of hours or days. Sustained release products can also be formulated for implantation or transdermal/transmucosal delivery. Such formulations typically will include a polymer that biodegrades or bioerodes thereby releasing a portion of the active ingredient. The formulations may have the form of microcapsules, liposomes, solid monolithic implants, gels, viscous fluids, discs, or adherent films.

Compressed tablets can be sugar coated or film coated to mask any unpleasant taste and protect the tablet from the atmosphere, or enteric coated for selective disintegration in the gastrointestinal tract.

Film-coated tablets are compressed tablets, which are covered with as thin layer of film or water-soluble material. A number of polymeric substances with film-forming properties may be used. Film coating imparts the same general characteristics as sugar coating with the added advantage of a greatly reduced time period required for the coating operation.

Enteric-coated tablets are compressed tablets coated with substances that resist solution in gastric fluid but disintegrate in the intestine. Enteric coatings can be used for tablets containing drug substances which are inactivated or destroyed in the stomach, for those which irritate the mucosa, or as a means of delayed release of the medication.

Multiple compressed tablets are compressed tablets made by more than one compression cycle.

Layered tablets are prepared by compressing additional tablet granulation on a previously compressed granulation. The operation my be repeated to produce multilayered tablets of two or three layers. Special tablet presses are required to make layered tablets.

Press-coated tablets, which are also referred to as dry-coated, are prepared by feeding previously compressed tablets into a special tableting machine and compressing another granulation layer around the preformed tablets. They have all the advantages of compressed tablets, i.e., slotting, monogramming, speed of disintegration, etc., while retaining the attributes of sugar-coated tablets in masking the taste of the drug substance in the core tablets. Press-coated tablets can also be used to separate incompatible drug substances; in addition, they can provide a means to give an enteric coating to the core tablets. Both types of multiple-compressed tablets have been widely used in the design of prolonged-action dosage forms.

Compressed tablets can be formulated to release the drug substance in a manner to provide medication over a period of time. There are a number of types which include delayed-action tablets in which the release of the drug substance is prevented for an interval of time after administration of until certain physiological conditions exist; repeat-action tablets which periodically release a complete dose of the drug substance to the gastrointestinal fluids; and the extended-release tablets which continuously release increments of the contained drug substance to the gastrointestinal fluids.

The non-aqueous carrier, or excipient, can be any substance that is biocompatible and liquid or soft enough at the mammal's body temperature to release the active ingredient into the animal's bloodstream at a desired rate. The carrier is usually hydrophobic and commonly organic, e.g., an oil or fat of vegetable, animal, mineral or synthetic origin or derivation. Preferably, but not necessarily, the carrier includes at least one chemical moiety of the kind that typifies "fatty" compounds, e.g., fatty acids, alcohols, esters, etc., i.e., a hydrocarbon chain, an ester linkage, or both. "Fatty" acids in this context include acetic, propionic and butyric acids through straight- or branched-chain organic acids containing up to 30 or more carbon atoms. Preferably, the carrier is immiscible in water and/or soluble in the substances commonly known as fat solvents. The carrier can correspond to a reaction product of such a "fatty" compound or compounds with a hydroxy compound, e.g., a mono-hydric, dihydric, trihydric or other polyhydric alcohol, e.g., glycerol, propanediol, lauryl alcohol, polyethylene or - propylene glycol, etc. These compounds include the fat-soluble vitamins, e.g., tocopherols and their esters, e.g., acetates sometimes produced to stabilize tocopherols. Sometimes, for economic reasons, the carrier may preferably comprise a natural, unmodified vegetable oil such as sesame oil, soybean oil, peanut oil, palm oil, or an unmodified fat. Alternatively the vegetable oil or fat may be modified by hydrogenation or other chemical means which is compatible with the present invention. The appropriate use of hydrophobic substances prepared by synthetic means is also envisioned.

Typically, water, suitable oil, saline, aqueous dextrose (glucose), and related sugar solutions and glycols such as propylene glycol or polyethylene glycols are suitable carriers for parenteral solutions. Solutions for parenteral administration preferably contain a water-soluble salt of the active ingredient, suitable stabilizing agents, and if necessary, buffer substances. Antioxidizing agents such as sodium bisulfite, sodium sulfite, or ascorbic acid, either alone or combined, are suitable stabilizing agents. Also used are citric acid and its salts, and sodium EDTA. In addition, parenteral solutions can contain preservatives, such as benzalkonium chloride, methyl- or propyl-paraben and chlorobutanol. Suitable pharmaceutical carriers are described in *Remington's Pharmaceutical Sciences, supra,* a standard reference text in this field.

In addition to the active or therapeutic ingredient, tablets contain a number of inert materials. The latter are known as additives or "adds." They may be classified according to the part they play in the finished tablet. The first group contains those which help to impart satisfactory compression characteristics to the formulation. These include (1) diluents, (2) binders, and (3) lubricants. The second group of added substances helps to give additional desirable physical characteristics to the finished tablet. Included in this group are (1) disintegrators, (2) colors, and in the case of chewable tablets, (3) flavors, and (4) sweetening agents.

Frequently the single dose of the active ingredient is small and an inert substance is added increase the bulk in order to make the tablet a practical size for compression. Diluents used for this purpose include dicalcium phosphate, calcium sulfate, lactose, kaolin, mannitol, sodium chloride, dry starch, and powdered sugar.

Most tablet formulators tend to use consistently only one or two diluents selected from the above group in their tablet formulations. Usually these have been selected on the basis of experience and cost factors. However, the compatibility of the diluent with the drug must be considered. When drug substances have low water solubility, it is recommended that water-soluble diluents be used to avoid possible bioavailability problems.

Agents used to impart cohesive qualities to the powdered material are referred to as binders or granulators. They impart a cohesiveness to the tablet formulation which insures the tablet remaining intact after compression, as well as improving the free-flowing qualities by the formulation of granules of desired hardness and size. Materials commonly used as binders include starch, gelatin, and sugars as sucrose, glucose, dextrose, molasses, and lactose. Natural and synthetic gums which have been used include acacia, sodium alginate, extract of Irish moss, panwar gum, ghatti gum, mucilage of isapol husks, carboxymethylcellulose, methylcellulose, polyvinylpyrrolidone, Beegum, and larch arabogalactan. Other agents which may be considered binders under certain circumstances are polyethylene glycol, ethylcellulose, waxes, water and alcohol.

The quality of binder used has considerable influence on the characteristics of the compressed tablets. The use of too much binder or too strong a binder will make a hard tablet which will not disintegrate easily. Alcohol and water are not binders in the true sense of the word; but because of their solvent action on some ingredients such as lactose and starch, they change the powdered material to granules and the residual moisture retained enables the materials to adhere together when compressed.

Lubricants have a number of functions in tablet manufacture. They improve the rate of flow of the tablet granulation, prevent adhesion of the tablet material to the surface of the dies and punches, reduce interparticle friction, and facilitate the ejection of the tablets from the die cavity. Commonly used lubricants include talc, magnesium stearate, calcium stearate, stearic acid, and hydrogenated vegetable oils. Most lubricants with the exception of talc are used in concentrations less than 1%. Lubricants are in most cases hydrophobic materials. Poor selection or excessive amounts can result in "waterproofing" the tablets, result in poor tablet disintegration and dissolution of the drug substance.

A disintegrator is a substance, or a mixture of substances, added to a tablet to facilitate its breakup or disintegration after administration. The active ingredient must be released from the tablet matrix as efficiently as possible to allow for its rapid dissolution. Materials serving as disintegrates have been chemically classified as starches, clays, celluloses, aligns, or gums.

The most popular disintegrators are corn and potato starch which have been well-dried and powdered. Starch has a great affinity for water and swells when moistened, thus facilitating the rupture of the tablet matrix. However, others have suggested that its disintegrating action in tablets is due to capillary action rather than swelling; the spherical shape of the starch grains increases the porosity of the tablet, thus promoting capillary action.

In addition to the starches a large variety of materials have been used and are reported to be effective as disintegrators. This group includes Veegum HV, methylcellulose, agar, bentonite, cellulose and wood products, natural sponge, cation-exchange resins, alginic acid, guar gum, citrus pulp, and carboxymethylcellulose. Sodium lauryl sulfate in combination with starch also has been demonstrated to be an effective disintegrant.

Colors in compressed tablets serve functions other than making the dosage from more esthetic in appearance. Any of the approved certified water-soluble FD&C dyes, mixtures of the same, or their corresponding lakes may be used to color tablets.

In addition to the sweetness which may be afforded by the diluent of the chewable tablet, e.g.. mannitol or lactose, artificial sweetening agents may be included. Among the most promising are two derivatives of glycyrrhizin, the glycoside obtained from licorice.

Compressed tablets may be characterized or described by a number of specifications. These include the diameter size, shape, thickness, weight, hardness, and disintegration time.

Useful pharmaceutical dosage-forms for administration of the compounds of this invention can be illustrated as follows:

### Capsules

A large number of unit capsules can be prepared by filling standard two-piece hard gelatin capsules each with 100 mg of powdered active ingredient, 150 mg of lactose, 50 mg of cellulose, and 6 mg magnesium stearic.

### Soft Gelatin Capsules

A mixture of active ingredient in digestible oil such as soybean oil, cottonseed oil or olive oil can be prepared and injected by means of a positive displacement pump into gelatin to form soft gelatin capsules containing 100 mg of the active ingredient. The capsules should then be washed and dried.

### Tablets

A large number of tablets can be prepared by conventional procedures so that the dosage unit is 100 mg of active ingredient, 0.2 mg of colloidal silicon dioxide, 5 milligrams of magnesium stearate, 275 mg of microcrystalline cellulose, 11 mg of starch and 98.8 mg of lactose.

### Suspension

An aqueous suspension can be prepared for oral administration so that each 5 mL contain 25 mg of finely divided active ingredient, 200 mg of sodium carboxymethyl cellulose, 5 mg of sodium benzoate, 1.0 g of sorbitol solution, U.S.P., and 0.025 mg of vanillin.

### Injectable

A parenteral composition suitable for administration by injection can be prepared by stirring 1.5% by weight of active ingredient in 10% by volume propylene glycol and water. The solution is sterilized by commonly used techniques.

### Combination of components (a) and (b)

Each therapeutic agent component useful in the present invention can independently be in any dosage form, such as those described above, and can also be administered in various ways, as described above. In the following description component (b) is to be understood to represent one or more agents as described previously. Thus, if components (a) and (b) are to be treated the same or independently, each agent of component (b) may also be treated the same or independently. Components (a) and (b) of the present invention may be formulated together, in a single dosage unit (that is, combined together in one capsule, tablet, powder, or liquid, etc.) as a combination product. When component (a) and (b) are not formulated together in a single dosage unit, the component (a) may be administered at the same time as component (b) or in any order; for example component (a) of this invention may be administered first, followed by administration of component (b), or they may be administered in the reverse order. If component (b) contains more that one agent, e.g., one antidepressant and one muscle relaxant, these agents may be administered together or separately in any order. When not administered at the same time, preferably the administration of component (a) and (b) occurs less than about one hour apart. Preferably, the route of administration of component (a) and (b) is oral.

The terms oral agent, oral compound, or the like, as used herein, denote compounds, which may be orally administered. Although it is preferable that component (a) and component (b) both be administered by the same route (that is, for example, both orally) or dosage form, if desired, they may each be administered by different routes (that is, for example, one component of the combination product may be administered orally, and another component may be administered intravenously) or dosage forms.

As is appreciated by a medical practitioner skilled in the art, the dosage of the combination therapy of the invention may vary depending upon various factors such as the pharmacodynamic characteristics of the particular agent and its mode and route of administration, the age, health and weight of the recipient, the nature and extent of the symptoms, the kind of concurrent treatment, the frequency of treatment, and the effect desired, as described above. The proper dosage of components (a) and (b) of the present invention will be readily ascertainable by a medical practitioner skilled in the art, based upon the present disclosure. By way of general guidance, typically a daily dosage may be about 100 milligrams to about 1.5 grams of each component. If component (b) represents more than one compound, then typically a daily dosage may be about 100 milligrams to about 1.5 grams of each agent of component (b). By way of general guidance, when the compounds of component (a) and component (b) are administered in combination, the dosage amount of each component may be reduced by about 70-80% relative to the usual dosage of the component when it is administered alone as a single agent for the treatment of irritable bowel syndrome in view of the synergistic effect of the combination.

The combination products of this invention may be formulated such that, although the active ingredients are combined in a single dosage unit, the physical contact between the active ingredients is minimized. In order to minimize contact, for example, where the product is orally administered, one active ingredient may be enteric coated. By enteric coating one of the active ingredients, it is possible not only to minimize the contact between the combined active ingredients, but also, it is possible to control the release of one of these components in the gastrointestinal tract such that one of these components is not released in the stomach but rather is released in the intestines. Another embodiment of this invention where oral administration is desired provides for a combination product wherein one of the active ingredients is coated with a sustained-release material which effects a sustained-release throughout the gastrointestinal tract and also serves to minimize physical contact between the combined active ingredients. Furthermore, the sustained-released component can be additionally enteric coated such that the release of this component occurs only in the intestine. Still another approach would involve the formulation of a combination product in which the one component is coated with a sustained and/or enteric release polymer, and the other component is also coated with a polymer such as a low-viscosity grade of hydroxypropyl methylcellulose or other appropriate materials as known in the art, in order to further separate the active components. The polymer coating serves to form an additional barrier to interaction with the other component. In each formulation wherein contact is prevented between components (a) and (b) via a coating or some other material, contact may also be prevented between the individual agents of component (b).

Dosage forms of the combination products of the present invention wherein one active ingredient is enteric coated can be in the form of tablets such that the enteric coated component and the other active ingredient are blended together and then compressed into a tablet or such that the enteric coated component is compressed into one tablet layer and the other active ingredient is compressed into an additional layer. Optionally, in order to further separate the two layers, one or more placebo layers may be present such that the placebo layer is between the layers of active ingredients. In addition, dosage forms of the present invention can be in the form of capsules wherein one active ingredient is compressed into a tablet or in the form of a plurality of microtablets, particles, granules or non-perils, which are then enteric coated. These enteric coated microtablets, particles, granules or non-perils are then placed into a capsule or compressed into a capsule along with a granulation of the other active ingredient.

These as well as other ways of minimizing contact between the components of combination products of the present invention, whether administered in a single dosage form or administered in separate forms but at the same time or concurrently by the same manner, will be readily apparent to those skilled in the art, based on the present disclosure.

Pharmaceutical kits useful for the treatment for visceral pain syndromes, and related diseases and symptoms, include a therapeutically effective amount of a pharmaceutical composition that includes a compound of component (a) and one or more compounds of component (b), in one or more sterile containers, Sterilization of the container may be carried out using conventional sterilization methodology well known to those skilled in the art. Component (a) and component (b) may be in the same sterile container or in separate sterile containers. The sterile containers of materials may comprise separate containers, or one or more multi-part containers, as desired. Component (a) and component (b), may be separate, or physically combined into a single dosage form or unit as described above. Such kits may further include, if desired, one or more of various conventional pharmaceutical kit components, such as for example, one or more pharmaceutically acceptable carriers, additional vials for mixing the components, etc., as will be readily apparent to those skilled in the art. Instructions, either as inserts or as labels, indicating quantities of the components to be administered, guidelines for administration, and/or guidelines for mixing the components, may also be included in the kit.

Various techniques are known to determine the norepinephrine (NE) - serotonin (5-HT) reuptake inhibition of a particular NSRI. In one embodiment, the ratio can be calculated from IC₅₀ data for NE and 5-HT reuptake inhibition. For example, it has been reported that for milnacipran the IC₅₀ of norepinephrine reuptake is 100 nM, whereas the IC₅₀ serotonin reuptake inhibition is 200 nM. See, Moret et al., Neuropharmacology, 29(12):1211-1219, 1985; Palmier, C., C. Puozzo, et al. (1989). "Monoamine uptake inhibition by plasma from healthy volunteers after single oral doses of the antidepressant milnacipran." Eur J Clin Pharmacol 37(3): 235-8.

The NE:5-HT reuptake inhibition ratio for milnacipran based on this data is 2:1. Other IC values such as IC₂₅, IC₇₅, etc. could be used, provided the same IC value is compared for both norepinephrine and serotonin. The concentrations necessary to achieve the desired degree of inhibition (i.e., IC value) can be calculated using known techniques either *in vivo* or in vitro. See, Sanchez, C. and J. Hyttel (1999). "Comparison of the effects of antidepressants and their metabolites on reuptake of biogenic amines and on receptor binding." Cell Mol Neurobiol 19(4): 467-89; Turcotte, J. E., G. Debonnel, et al. (2001). "Assessment of the serotonin and norepinephrine reuptake blocking properties of duloxetine in healthy subjects." Neuropsychopharmacology 24(5): 511-21; Moret, C., M. Charveron, et al. (1985). "Biochemical profile of midalcipran (F 2207), 1-phenyl-1-diethyl- aminocarbonyl-2-aminomethyl-cyclopropane (Z) hydrochloride, a potential fourth generation antidepressant drug." Neuropharmacology 24(12): 1211-9; Moret, C. and M. Briley (1997). "Effects of milnacipran and pindolol on extracellular noradrenaline and serotonin levels in guinea pig hypothalamus." J Neurochem 69(2): 815-22; Bel, N. and F. Artigas (1999). "Modulation of the extracellular 5-hydroxytryptamine brain concentrations by the serotonin and noradrenaline reuptake inhibitor, milnacipran. Microdialysis studies in rats." Neuropsychopharmacology 21(6): 745-54; and Palmier, C., C. Puozzo, et al. (1989). "Monoamine uptake inhibition by plasma from healthy volunteers after single oral doses of the antidepressant milnacipran." Eur J Clin Pharmacol 37(3): 235-8.

The following examples are introduced in order that the invention may be more readily understood.

### Examples

### Example 1. Efficacy of Milnacipran in the treatment of Irritable Bowel Syndrome

### METHODS:

A 12-week dose escalation monotherapy trial was conducted to evaluate milnacipran's efficacy in patients with a diagnosis of Irritable Bowel Syndrome (IBS) comorbid with fibromyalgia. Patients were washed off of a variety medications-including centrally acting stimulants, antidepressants and sedative-hypnotics-over a 2-4 week period; this was followed by a two-week baseline period. After successful completion of the baseline period, patients were started on milnacipran. All patients were started at a dose of 25mg daily, and were then escalated weekly over a 4-week period to 50, 100, and finally 200 mg daily, or until dose-limiting toxicity (DLT) was evident.
In the event that DLT was evident, the patient was stabilized at the previously well-tolerated dosage, and remained on this dose for eight weeks at stable dose therapy.

The patient global impression of change (PGIC) was administered at clinic visits scheduled during the 4^{th}, 8^{th}, and 12^{th} weeks of treatment. The PGIC is a highly clinically relevant instrument useful for measurement of patient improvement during controlled clinical trials. (Indeed, this instrument was the basis by which alosetron (Lotronex™) was approved by the Food and Drug Administration for the IBS indication.)

The PGIC is a conceptually simple instrument which assesses the patient's overall satisfaction with a course of drug therapy. This is done by asking the patient, "Since the start of the study, my overall status is:" with the patient choosing from the 7 choices depicted in Table 1.

**Table 1**

| | |
|---|---|
| 1. | Very much improved |
| 2. | Much improved |
| 3. | Minimally improved |
| 4. | No change |
| 5. | Minimally worse |
| 6. | Much worse |
| 7. | Very much worse |

For the present purposes, responses between 1-3 and 5-7 were collapsed into "Improved" and "Worse", respectively.

### RESULTS:

11 subjects were met the criteria of a history of IBS in the context of fibromyalgia and completed the trial. Figure 1 summarizes the PGIC scores of these 11 patients collected at their 12 week clinic visits.

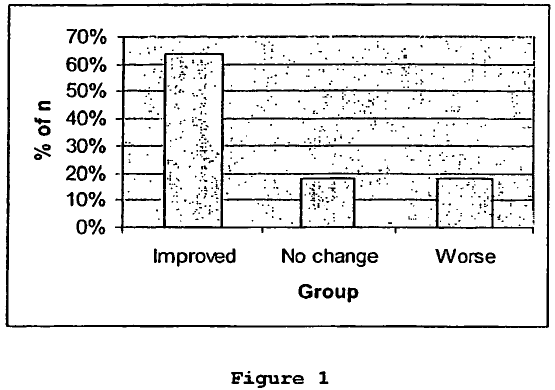

Importantly, there were no serious adverse events during the course of the trial. All milnacipran related adverse events within this group were transitory in nature with the most common being intermittent nausea. This group also had a low rate of constipation with only one reported AE.

### CONCLUSION:

This trial demonstrates that milnacipran is a safe and effective therapy for IBS.

The following prophetic examples illustrate orally administered solid dosage formulations that can be prepared to include the active ingredient.

### Example 2

The active ingredient can be prepared as a controlled release pharmaceutical composition, as described in U.S. Patent No. 6,991,950; wherein the active ingredient as described herein is substituted for the active ingredient described therein. The composition can include a matrix of a material that includes a high melting point fatty acid ester, an oil, a polymeric cellulose derivative, or a combination thereof. The active ingredient can optionally be associated with the matrix. The formulation can optionally include a surfactant (e.g., polysorbate 80).

Suitable high melting fatty acid esters include, e.g., glyceryl behenate, glyceryl palmitostearate, and glyceryl stearate. Suitable oils include, e.g., corn oil, cottonseed oil, menhaden oil, safflower oil, sesame oil, shark-liver oil, soybean oil, olive oil, and wheat germ oil. Suitable cellulosic polymers include, e.g., a low-substituted hydroxypropyl ether cellulose polymer and a cellulosic polymer having methylether substitution. Suitable high melting fatty acid esters include, e.g., glyceryl behenate, glyceryl palmitostearate and glyceryl stearate.

Additional substances that can be included in the above pharmaceutical compositions, as well as methods to make the pharmaceutical compositions, are described in U.S. Patent No. 6,491,950.

### Example 3

The active ingredient can be prepared as a biphasic controlled release pharmaceutical composition, as described in U.S. Patent No. 6,475,521; wherein the active ingredient as described herein is substituted for the active ingredient described therein. Such a system can provide a dosage form that has prolonged gastric residence so that the active ingredient can be administered once daily to sustain a continuous plasma concentration of the active ingredient.

The controlled release pharmaceutical composition includes an inner solid particulate phase formed of substantially uniform granules containing the active ingredient, one or more hydrophilic polymers, and one or more hydrophobic polymers. The delivery system can also include one or more hydrophobic materials, such as one or more waxes, fatty alcohols and/or fatty acid esters. The controlled release pharmaceutical composition has an outer solid continuous phase in which the above granules of inner solid particulate phase are embedded and dispersed throughout. This outer solid continuous phase includes one or more hydrophilic polymers, one or more hydrophobic polymers and/or one or more hydrophobic materials such as one or more waxes, fatty alcohols and/or fatty acid esters. The controlled release pharmaceutical composition may be compressed into tablets or filled into capsules.

The particles of the inner solid particulate phase can include the active ingredient and an extended release material. The outer solid continuous phase can include an extended release material.

Additional substances that can be included in the above pharmaceutical compositions, as well as methods to make the pharmaceutical compositions, are described in U.S. Patent No. 6,475,521.

### Example 4

The active ingredient can be prepared as a controlled release tablet form, having a hydrophilic matrix that is suitable for the once-a-day administration, as described in U.S. Patent No. 6,419,953; wherein the active ingredient of the present invention is substituted for the active ingredient described therein. The tablet can include from about 50 weight percent to about 55 weight percent of the active ingredient, from about 20 weight percent to about 40 weight percent hydroxypropyl methylcellulose, from about 5 weight percent to about 15 weight percent lactose, from about 4 weight percent to about 6 weight percent microcrystalline cellulose, and from about 1 weight percent to about 5 weight percent of silicon dioxide. All of the weight percentages are based upon the total weight of the tablet dosage form.

More specifically, the controlled release tablet can be formed from a uniform admixture of about 54 weight percent of the active ingredient, about 30 weight percent hydroxypropyl methylcellulose, about 8 weight percent lactose, about 5 weight percent microcrystalline cellulose, and about 3 weight percent silicon dioxide.

More specifically, the controlled release tablet can also be formed from a uniform admixture of about 54 weight percent of the active ingredient, about 30 weight percent hydroxypropyl methylcellulose, about 8 percent lactose, about 5 weight percent microcrystalline cellulose, and about 3 weight percent silicon dioxide.

Additional substances that can be included in the above controlled release tablets, as well as methods to make the controlled release tablets, are described in U.S. Patent No. 6,419,953.

### Example 5

The active ingredient can be prepared as a controlled release gelatin capsule formed with a composite wall that contains a liquid, the active ingredient formulation, as described in U.S. Patent No. 6,419,952; wherein the active ingredient as described herein is substituted for the active ingredient described therein. The composite wall includes a barrier layer formed over the external surface of the gelatin capsule, an expandable layer formed over the barrier layer, and a semipermeable layer formed over the expandable layer.

The controlled release gelatin capsule includes a gelatin capsule containing a liquid, the active ingredient formulation; and a multilayer wall superposed on the gelatin capsule. The multilayer wall includes a deformable barrier layer, an expandable layer, a semipermeable layer; and an orifice formed or formable through the wall.

Additional substances that can be included in the above controlled release gelatin capsules, as well as methods to make the controlled release gelatin capsules, are described in U.S. Patent No. 6,419,952.

### Example 6

The active ingredient can be prepared as a sustained-release dosage form having the active ingredient surrounded by an interior and an exterior wall, with an exit that allows for administration of the active ingredient to a patient, as described in U.S. Patent No. 6,245,357; wherein the active ingredient as described herein is substituted for the active ingredient described therein.

The sustained-release dosage form can include the active ingredient, and a pharmaceutically acceptable polyethylene oxide carrier, which is coated with a wall comprising ethylcellulose and hydroxypropylcellulose.

More specifically, the sustained-release dosage form can also include the active ingredient and a pharmaceutically acceptable polyethylene oxide carrier, which is coated with an interior wall comprising ethyl cellulose and hydroxypropylcellulose, and an exterior wall containing cellulose acetate.

The sustained-release dosage form can also be prepared as a dosage form for delivering the active ingredient at a sustained-release rate to a gastrointestinal-lipid-fluid environment. The dosage form includes a composition containing a dose of the active ingredient, and a coat that envelopes the composition containing the active ingredient. The coat includes a passage-former that leaves the coat in the presence of fluid, and a wall that surrounds the coat and prevents lipid in the gastrointestinal tract from entering the dosage form.

Additional substances that can be included in the above sustained-release dosage forms, as well as methods to make the sustained-release dosage forms, are described in U.S. Patent No. 6,245,357.

### Example 7

The active ingredient can be prepared as a tablet for controlled release, as described in U.S. Patent No. 6,033,685; wherein the active ingredient as described herein is substituted for the active ingredient described therein. The tablet includes a matrix layer having the active ingredient embedded in a non-swelling, non-gelling hydrophobic matrix; a first barrier layer laminated to a single face of the matrix layer; and an optional second barrier layer laminated to the opposite face of the matrix layer and oppositely disposed to the first barrier layer. The matrix contains up to about 80% of the active ingredient, and from about 5% to about 80% by weight of nonswellable waxes or polymeric material insoluble in aqueous medium. The first and second barrier layers independently include polymeric material exhibiting a high degree of swelling and gelling in aqueous medium, or nonswellable wax or polymeric material insoluble in aqueous medium.

Additional substances that can be included in the above controlled release tablets, as well as methods to make the controlled release tablets, are described in U.S. Patent No. 6,033,685.

### Example 8

The active ingredient can be prepared as a pharmaceutical composition for extended release of the active ingredient in a gastrointestinal environment, as described in U.S. Patent No. 6,010,718; wherein the active ingredient as described herein is substituted for the active ingredient described therein. The composition includes the active ingredient and a pharmaceutically acceptable polymer so that, when ingested orally, the composition induces statistically significantly lower Cₘₐₓ in the plasma than an immediate release composition of the active ingredient. The pharmaceutical composition maintains bioavailability and minimum concentration substantially equivalent to that of an immediate release composition of the active ingredient achieved by multiple dosing.

Additional substances that can be included in the above extended release pharmaceutical compositions, as well as methods to make the extended release pharmaceutical compositions, are described in U.S. Patent No. 6,010,718.

### Example 9

The active ingredient can be prepared as orally administrable pharmaceutical preparations having controlled release of the active ingredient, as described in U.S. Patent No. 5,900,425; wherein the active ingredient as described herein is substituted for the active ingredient described therein. Such controlled release pharmaceutical preparations can include the active ingredient in amorphous form as a coprecipitate in a polyvinylpyrrolidone homo or copolymer having a weight average molecular weight of about 15,000 to 1,000,000 and, a release-delaying component containing a gel-forming polymer having a viscosity of at least 15 mPas when measured at a 2% concentration at 20°C.

Additional substances that can be included in the orally administrable extended release pharmaceutical compositions, as well as methods to make the orally administrable extended release pharmaceutical compositions are described in U.S. Patent No. 5,900,425.

### Example 10

The active ingredient can be prepared in tablet form for controlled release of the active ingredient in a dispersion as described in U.S. Patent No. 5,882,682; wherein the active ingredient as described herein is substituted for the active ingredient described therein. The tablet has a compressed core which contains the active agent, a polymer which forms gelatinous microscopic particles upon hydration, and if desired, an agent to modulate the hydration; and a water insoluble coating which adheres to and surrounds the core and contains apertures which provide an area for the hydration and release of the dispersion. The release rate of the active ingredient is a function of the number and size of the apertures in the coating of the tablet.

The active ingredient may be prepared for controlled release from a tablet as a dispersion by preparing a compressed core from an admixture containing a therapeutically effective amount of the active ingredient, a polymer which upon hydration forms gelatinous microscopic particles, and a water insoluble, water impermeable polymeric coating.

The water insoluble, water impermeable polymeric coating can contain a polymer and a plasticizer, which surrounds and adheres to the core. The polymer can include, e.g., cellulose acetate, cellulose acetate butyrate, ethylcellulose, polyvinylacetate, polyvinyl chloride, polymers of acrylic, methacrylic acid esters, or a combination thereof. The plasticizer can include, e.g., dibutylsebacate, diethylphthalate, triethylcitrate, polyethylene glycol, or a combination thereof. The polymer which upon hydration forms gelatinous microscopic particles can include, e.g., sodium polyacrylate, carboxypolymethylenes, the pharmaceutically acceptable salts thereof, or a combination thereof. The carboxypolymethylenes can be prepared from acrylic acid crosslinked with allylethers of sucrose or pentaerythritol. The coating of the tablet can have a plurality of formed apertures exposing between about 1 and about 75% of the core surface.

Additional substances that can be included in the orally administrable tablets for the controlled release of the active ingredient in a dispersion, as well as methods to make the orally administrable tablets for the controlled release of the active ingredient in a dispersion are described in U.S. Patent No. 5,882,682.

### Example 11

The active ingredient can be prepared as a tablet for controlled release of the active ingredient through use of a water-soluble alginate salt, a complex salt of alginic acid and an organic carboxylic acid in admixture with the active ingredient, as described in U.S. Patent No. 5,705,190; wherein the active ingredient as described herein is substituted for the active ingredient described therein.

A tablet for a once a day dosage of the active ingredient can be prepared that contains a therapeutically effective amount of the active ingredient, a water-soluble alginate salt, a complex salt of alginic acid, and an organic carboxylic acid. The cation of the alginic acid can be calcium, strontium, iron, or barium.

Additional substances that can be included in the orally administrable controlled release tablets, as well as methods to make the orally administrable controlled release tablets are described in U.S. Patent No. 5,705,190.

### Example 12

An oral composition of the active ingredient can be prepared for targeted slow release of the active ingredient in the intestine, as described in U.S. Patent No. 5,643,602; wherein the active ingredient as described herein is substituted for the active ingredient described therein.

Oral compositions can be prepared that contain the active ingredient in a pellet that contains a core, a layer that surrounds the core, and a membrane that surrounds the layer and the core. The core can contain the active ingredient alone or in combination with other pharmaceutically acceptable materials. The layer surrounding the core can be a pharmaceutically acceptable film-forming, water-insoluble or water-soluble polymer; a pharmaceutically acceptable mixture of film-forming, water-insoluble polymers; or a pharmaceutically acceptable mixture of film-forming, water-soluble and film-forming, water-insoluble polymers.

The membrane surrounding both the core and the layer surrounding the core can contain a pharmaceutically acceptable, film-forming, anionic carboxylic polymer that is difficult to dissolve at a low pH but that is soluble at a higher pH of about 4 to 7.5. The polymer of the membrane can be either alone or in combination with a pharmaceutically acceptable, film-forming, water-insoluble polymer. The thickness or the ratio of the anionic carboxylic polymer to the water-insoluble polymer is effective to prevent release of the active ingredient from the pellet in gastric fluids, but permits release of the active ingredient from the pellet in intestinal fluids at a rate allowing treatment of a part of the intestinal tract.

Additional substances that can be included in the orally administrable controlled release tablets that can be targeted to the intestine, as well as methods to make the orally administrable controlled release tablets that can be targeted to the intestine are described in U.S. Patent No. 5,643,602.

### Example 13

A sustained release once-a-day oral formulation of the active ingredient can be prepared that contains a therapeutically effective amount of the active ingredient and a non-aqueous semisolid matrix to impart sustained release properties to the active ingredient, as described in U.S. Patent No. 5,433,951; wherein the active ingredient as described herein is substituted for the active ingredient described therein. The non-aqueous semisolid matrix is a fatty acid glyceride and/or a polyethylene glycol ester of a fatty acid. The semisolid matrix can be a long chain fatty acid glycerides and/or one or a mixture of polyethylene glycol esters of long chain fatty acids, and mixtures thereof.

Additional substances that can be included in the orally administrable sustained release tablets, as well as methods to make the orally administrable sustained release tablets are described in U.S. Patent No. 5,433,951.

### Example 14

The active ingredient can be prepared as an orally administrable formulation that contains the active ingredient and a permeation-enhancing mixture of sodium salicylate and an oil to provide enhanced absorption of the active ingredient through the wall of the gastrointestinal tract, as described in U.S. Patent No. 5,424,289; wherein the active ingredient as described herein is substituted for the active ingredient described therein. The formulation is characterized as a solid, which provides a convenient and improved format for handling and storage and for the preparation of oral dosage forms (such as pills, capsules and delivery vessels) containing a homogeneous mixture of ingredients.

The active ingredient can be prepared as a dosage form having an orally administrable, enteric-coated capsule that contains a therapeutically effective amount of the active ingredient, 70-90 weight % of sodium salicylate, and 10-30 weight % of an oil.

Additional substances that can be included in the orally administrable tablets, as well as methods to make the orally administrable tablets are described in U.S. Patent No. 5,424,289.

### Example 15

The active ingredient can be prepared as oral controlled release dosage units that contain hydroxypropyl methylcellulose, as described in U.S. Patent No. 5,419,918; wherein the active ingredient as described herein is substituted for the active ingredient described therein. The aqueous granulation of the dosage units is performed in the presence of one or more solutes, which inhibit gel formation during granulation, but allow formation of a gel when administered orally.

Additional substances that can be included in the orally administrable dosage units, as well as methods to make the orally administrable dosage units are described in U.S. Patent No. 5,419,918.

### Example 16

The active ingredient can be prepared as a mixture of an alginate and a polyacrylate in a ratio of from 15:1 to 1:2, as described in U.S. Patent No. 5,230,901; wherein the active ingredient as described herein is substituted for the active ingredient described therein. Such mixtures are suitable for the preparation of depot drug forms.

The active ingredient may be prepared as a tablet for sustained release that includes a blend of a unit dosage of the active ingredient with a mixture of alginate and a polyacrylate in a ratio of 15:1 to 2:1. The polyacrylate can be a copolymer of neutral (meth)acrylic acid esters of methanol, ethanol and trimethylammonioethanol chloride. In addition, the ratio of the ammonium group containing ester unit to the remaining neutral (meth)acrylic acid ester units can be about 1:40.

Additional substances that can be included in the tablets, as well as methods to make the tablets, are described in U.S. Patent No. 5,230,901.

### Example 17

The active ingredient can be prepared as a controlled release pellet containing a core which includes the active ingredient, an intensive disintegrating agent, a wetting agent and a binder; and a double layer which controls release of the activate agent, as described in U.S. Patent No. 5,204,121; wherein the active ingredient as described herein is substituted for the active ingredient described therein. The double layer includes an acrylic-based outer undigestible water-permeable lacquer layer, and an inner jacket layer that contains a hydrophobic additive and hydroxypropylcellulose. The intensive disintegrating agent can be crosslinked sodium carboxymethylcellulose or sodium starch glycolate. The wetting agent can include sodium laurylsulphate. The binder can include PVP. The outer undigestible water-permeable lacquer layer can include an acrylic resin based on a poly(meth)acrylic acid ester having a neutral character or having a low content of quaternary ammonium groups. Such an acid ester can include a copoly(meth)acrylic acid ester, or an ethylcellulose. The inner jacket controls the migration of the water in the direction of the core. The inner jacket can contain hydroxypropylcellulose and a hydrophobic additive that is calcium stearate or hydrogenated castor oil.

Additional substances that can be included in the tablets, as well as methods to make the tablets, are described in U.S. Patent No. 5,204,121.

### Example 18

The active ingredient can be prepared as a sustained release formulation containing the active ingredient and a high and low viscosity HPMC, as described in U.S. Patent No. 5,009,895; wherein the active ingredient as described herein is substituted for the active ingredient described therein. The sustained release formulation will exhibit a zero order release profile.

A carrier base material can be combined with the active ingredient and shaped and compressed to a solid sustained release pharmaceutical dosage form having a zero order release profile upon administration. The carrier base material can contain a high viscosity hydroxymethylpropylcellulose (HPMC) having a molecular weight of 60,000 or greater; and a low viscosity HPMC, having a molecular weight of 50,000 or less. The high and low viscosity HPMC are in a ratio yielding a zero order release profile.

Additional substances that can be included in the sustained release formulations, as well as methods to make the sustained release formulations, are described in U.S. Patent No. 5,009,895.

### Example 19

The active ingredient can be prepared as a controlled and sustained release formulation containing a carrier base material combined with the active ingredient, as described in U.S. Patent No. 4,983,398; wherein the active ingredient as described herein is substituted for the active ingredient described therein. The carrier base material can contain a mixture of one or more nonionic cellulose ethers and an alkali metal carboxylate. At least one of the cellulose ethers can include hydroxypropylmethylcellulose having a number average molecular weight of at least 50,000.

Additional substances that can be included in the sustained release formulations, as well as methods to make the sustained release formulations, are described in U.S. Patent No. 4,983,398.

### Example 20

The active ingredient can be prepared as a controlled release formulation for the controlled release of the active ingredient, as described in U.S. Patent No. 4,946,686; wherein the active ingredient as described herein is substituted for the active ingredient described therein. The formulation includes a core composition containing a plurality of controlled release solubility modulating units that include solubility modulating agents. Each solubility modulating agent is a complexing agent or a surfactant, and is either surrounded by a water insoluble coat containing at least one pore forming additive dispersed throughout, or dispersed in an individual matrix substrate. Each unit also includes the active ingredient, and a water insoluble microporous wall that surrounds the core composition. The water insoluble microporous wall contains a polymer material that is permeable to water but substantially impermeable to solute, and at least one water leachable pore forming additive dispersed throughout the wall.

Additional substances that can be included in the controlled release formulations, as well as methods to make the controlled release formulations, are described in U.S. Patent No. 4,946,686.

### Example 21

The active ingredient can be prepared as an oral sustained release tablet having a core and a coating layer, as described in described in U.S. Patent No. 4,919,938; wherein the active ingredient as described herein is substituted for the active ingredient described therein. The core matrix can contain 20% to 60% w/w of a hydroxypropylmethylcellulose gelling agent, 0.41% to 20% w/w of (+)-trans-1a,2,3,9a,5,6-hexahydro-9-hydroxy-4-(1-propyl)-4H-naphth[1,2-b]- 1,4-oxazine hydrochloride, and 2.08 to 12.5% w/w of buffering agent homogeneously dispersed therein. The core can also include suitable pharmaceutically acceptable excipients. The coating layer surrounding the core matrix can include a slowly soluble, water permeable ethyl cellulose polymer.

Additional substances that can be included in the controlled release tablets, as well as methods to make the controlled release tablets, are described in U.S. Patent No. 4,919,938.

### Example 22

The active ingredient can be prepared as a solid unit dosage form having a controlled and prolonged release pattern upon administration, as described in U.S. Patent No. 4,849,229; wherein the active ingredient as described herein is substituted for the active ingredient described therein. The dosage form can contain a mixture of a high viscosity grade methylcellulose or hydroxypropylmethylcellulose, an alkali metal sulfate or sulfonate and the active ingredient.

A therapeutically active solid unit dosage form having a controlled and prolonged release pattern upon administration, can contain a mixture of a high viscosity grade water-soluble nonionic cellulose ether having a number average molecular weight of at least 50,000 and a methoxyl content of 16.5-31.5 weight-%. The cellulose ether can include methylcellulose, hydroxypropylmethylcellulose, or mixtures thereof. The dosage form can also include an alkali metal sulfonate of aliphatic and aromatic hydrocarbons and succinic esters, and the active ingredient.

Additional substances that can be included in the dosage form, as well as methods to make the dosage form, are described in U.S. Patent No. 4,849,229.

### Example 23

The active ingredient can be prepared as a controlled, slow release, solid pharmaceutical composition that includes the active ingredient and a blend of sodium alginate and sodium-calcium alginate, as described in U.S. Patent No. 4,842,866; wherein the active ingredient as described herein is substituted for the active ingredient described therein.

Additional substances that can be included in the dosage form, as well as methods to make the dosage form, are described in U.S. Patent No. 4,842,866.

### Example 24

The active ingredient can be prepared as a controlled and prolonged release composition having a carrier base material that is combined with the active ingredient and shaped and compressed to a solid unit dosage form, as described in U.S. Patent No. 4,795,327; wherein the active ingredient as described herein is substituted for the active ingredient described therein. The carrier base material is a mixture of one or more nonionic cellulose ethers and an anionic surfactant. At least one of the cellulose ethers is methyl cellulose or hydroxypropylmethylcellulose having a number average molecular weight of at least 50,000 and a methoxyl content of 16.5-31.5 weight-%.

Additional substances that can be included in the dosage form, as well as methods to make the dosage form, are described in U.S. Patent No. 4,795,327.

### Example 25

The active ingredient can be prepared as a hydrogel reservoir containing pills that provide for controlled delivery of the active ingredient, as described in U.S. Patent No. 4,649,043; wherein the active ingredient as described herein is substituted for the active ingredient described therein. The pills include a wall surrounding a core of the active ingredient.

The hydrogel reservoir includes a matrix that contains a pharmaceutically acceptable non-toxic, non-hydrated polyethylene oxide that exhibits the ability to retain fluid within its polyethylene oxide structure, absorb fluid from the gastrointestinal tract, and expand with at least a 2 fold volume increase for retaining the hydrogel reservoir in the stomach over an extended period of time. The hydrogel reservoir includes a plurality of pills dispensed throughout the matrix of the reservoir. The pills contain a dosage amount of the active ingredient and a wall containing a release rate controlling composition that contains a cellulosic polymer that surrounds the dosage amount of the active ingredient. The matrix can contain a pharmaceutically acceptable non-toxic, non-hydrated carboxy polymer that exhibits the ability to retain fluid within its carboxy polymer structure, absorb fluid from the gastrointestinal tract, and expand with at least a 2 fold volume increase for retaining the dispensing device in the stomach over an extended period of time.

Additional substances that can be included in the hydrogel reservoirs, as well as methods to make the hydrogel reservoirs, are described in U.S. Patent No. 4,649,043.

### Example 26

The active ingredient can be prepared as a sustained release composition that is made from a plurality of pellets, as described in U.S. Patent No. 4,634,587; wherein the active ingredient as described herein is substituted for the active ingredient described therein. Each pellet can include the active ingredient-containing coating over a nonpareil seed, with a further coating of about 5 to about 15% by weight of a mixture of about 1.5 to about 9 parts by weight ethylcellulose to about 1 part by weight hydroxypropylcellulose.

Additional substances that can be included in the sustained release compositions, as well as methods to make the sustained release compositions, are described in U.S. Patent No. 4,634,587.

### Example 27

The active ingredient can be prepared as a sustained release oral formulation that contains a capsule that includes upper and lower parts that are connectible and easily separable from each other, and a plurality of micropellets present in the capsule, as described in U.S. Patent No. 4,587,118; wherein the active ingredient as described herein is substituted for the active ingredient described therein. The micropellets provide sustained release of the active ingredient when taken by a patient. The micropellets contain inner seeds coated with a mixture of theophylline and polyvinylpyrrolidone which is further coated with a mixture of ethylcellulose and hydroxypropylcellulose.

Additional substances that can be included in the sustained release compositions, as well as methods to make the sustained release compositions, are described in U.S. Patent No. 4,587,118.

### Example 28

The active ingredient can be prepared as a sustained release tablet for oral administration, as described in U.S. Patent No. 4,556,678; wherein the active ingredient as described herein is substituted for the active ingredient described therein. The tablet contains compressed granules that include the active ingredient, from about 0.1 to about 10 parts by weight hydroxypropyl methylcellulose, about one part by weight hydroxypropyl cellulose, and a lubricant. The hydroxypropyl methylcellulose will have a molecular weight of from about 20,000 to about 140,000. The hydroxypropyl cellulose will have a molecular weight of from about 60,000 to about 300,000.

Additional substances that can be included in the sustained release compositions, as well as methods to make the sustained release compositions, are described in U.S. Patent No. 4,556,678.

### Example 29

The active ingredient can be prepared as an oral unit dosage containing a carrier base material and the active ingredient for controlled and prolonged release, as described in U.S. Patent No. 4,540,566; wherein the active ingredient as described herein is substituted for the active ingredient described therein. The carrier base material can be a mixture of one or more nonionic cellulose ethers and an anionic surfactant. At least one of the cellulose ethers can be a modified hydroxypropylmethylcellulose having a number average molecular weight of less than 50,000 and has been modified by successive or concurrent exposure to moisture and air.

Additional substances that can be included in the sustained release compositions, as well as methods to make the sustained release compositions, are described in U.S. Patent No. 4,540,566.

### Example 30

The active ingredient can be prepared as a sustained release composition that contains a plurality of polymerically coated seeds of the active ingredient, as described in U.S. Patent No. 4,508,702; wherein the active ingredient as described herein is substituted for the active ingredient described therein. Each of the seeds can be individually coated with a polymeric mixture, which contains from about 1.5 to about 15 parts by weight ethylcellulose and about one part by weight hydroxypropylcellulose.

Additional substances that can be included in the sustained release compositions, as well as methods to make the sustained release compositions, are described in U.S. Patent No. 4,508,702.

### Example 31

The active ingredient can be prepared as a self-supporting polymeric diffusion matrix that provides for the sustained release of the active ingredient, as described in U.S. Patent No. 4,482,533; wherein the active ingredient as described herein is substituted for the active ingredient described therein. The matrix can contain from about 1 to about 60% by weight of a polar plasticizer; from about 5 to about 20% by weight polyvinylalcohol having a molecular weight from about 50,000 to about 150,000; from about 10 to about 25% by weight polyvinylalcohol having a molecular weight from about 4,000 to about 15,000; from about 2 to about 30% by weight polyvinylpyrrolidone; a pharmaceutically effective amount of the active ingredient to provide a sustained release of the active ingredient over a prolonged period; and from about 5 to about 20% by weight of diethanol myristoylamide. The diethanol myristoylamide can function to bring the components into solution.

Additional substances that can be included in the sustained release matrixes, as well as methods to make the sustained release matrixes, are described in U.S. Patent No. 4,482,533.

### Example 32

The active ingredient can be prepared as a sustained release oral dosage form as a tablet having a core that contains a pharmaceutically effective amount of the active ingredient, as described in U.S. Patent No. 4,432,965; wherein the active ingredient as described herein is substituted for the active ingredient described therein. The tablet core can be coated with a sustained release polymeric coating which contains about 5 to about 20 percent by weight polyethylene glycol component having a molecular weight of from about 500 to about 2000, and from about 80 to 95 percent by weight polyvinylalcohol component. The polyvinylalcohol component can contain from about one to about ten parts by weight of a partially hydrolyzed polyvinylalcohol subcomponent having a molecular weight of from about 50,000 to about 110,000 and having a degree of hydrolysis of from about 75 to about 92 percent. The polyvinylalcohol component can also contain about one part by weight of a substantially completely hydrolyzed polyvinylalcohol subcomponent having a molecular weight of from about 90,000 to about 150,000 and having a degree of hydrolysis in excess of 95%.

Additional substances that can be included in the sustained release oral dosage forms, as well as methods to make the sustained release oral dosage forms, are described in U.S. Patent No. 4,432,965.

### Example 33

The active ingredient can be prepared as pharmaceutical tablets, lozenges, suppositories and other solid dosage unit forms that have a prolonged and regular release pattern of the active ingredient, as described in U.S. Patent No. 4,226,849; wherein the active ingredient as described herein is substituted for the active ingredient described therein. The shaped dosage unit can contain a carrier base material of hydroxypropylmethylcellulose or a mixture thereof with up to 30% ethylcellulose and/or up to 30% sodium carboxymethylcellulose. The carrier base material can be subjected to hydrolysis and oxidation, so as to generate a desired minimum concentration of carbonyl and carboxyl groups, and then admixed and shaped with the active ingredient of the invention.

Additional substances that can be included in the sustained release dosage forms, as well as methods to make the sustained release dosage forms, are described in U.S. Patent No. 4,226,849.

### Example 34

The active ingredient can be prepared as a sustained release composition that utilizes a pellet formulation encapsulated in a hard gelatin capsule, as described in U.S. Patent No. 4,173,626; wherein the active ingredient as described herein is substituted for the active ingredient described therein. A portion of the pellets can be uncoated for immediate and rapid release of the active ingredient for elevating the plasma level of the active ingredient. The remainder of the pellets can be coated with a polymer to sustain the plasma level of the active ingredient. The uncoated and coated pellets may be mixed with non-medicated pellets as a capsule filler.

Additional substances that can be included in the sustained release compositions, as well as methods to make the sustained release compositions, are described in U.S. Patent No. 4, 73,626.

### Example 35

The active ingredient can be prepared as a controlled release solid dosage composition, as described in U.S. Patent No. 6,365,196; wherein the active ingredient as described herein is substituted for the active ingredient described therein. The composition can include a dissolution rate stabilizer and a hydrophobic waxy material. The composition can contain about 40 to 90% by weight of the active ingredient, a hydrophobic waxy material in about 5 to 30% by weight, a dissolution rate stabilizer in an amount greater than 1% to about 15% by weight; and optional pharmaceutically acceptable excipients.

Additional substances that can be included in the above compositions, as well as methods to make the compositions are described in U.S. Patent No. 6,365,196.

### Example 36

The active ingredient can be prepared as a stabilized solid controlled release dosage form, as described in U.S. Patent No. 6,316,031; wherein the active ingredient as described herein is substituted for the active ingredient described therein. The controlled release dosage form can have an inert bead coated with the active ingredient, a barrier layer over the bead that is coated with the active ingredient, and a controlled release layer that is added over the barrier layer.

The barrier layer can include hydroxypropylmethylcellulose. The barrier layer can be coated with a controlled release layer derived from an aqueous dispersion of plasticized ethylcellulose in an amount sufficient to obtain controlled release of the active ingredient when the bead is exposed to a gastrointestinal fluid. The coated bead will be cured at a temperature greater than the glass transition temperature of the plasticized ethylcellulose for at least about 24 hours. This will cause individual ethylcellulose particles in the coating to coalesce and to gradually slow the release of the active ingredient when the bead is exposed to aqueous fluid until an endpoint is reached. When the endpoint is reached, the active ingredient will be released in amounts which do not significantly vary at any time point along the dissolution curve by more than about 20% of the total amount of the active ingredient released, when compared to the in-vitro dissolution of the coated bead prior to curing.

Additional substances that can be included in the above compositions, as well as methods to make the compositions are described in U.S. Patent No. 6,316,031.

### Example 37

The active ingredient can be prepared as a stable solid controlled release composition, as described in U.S. Patent No. 6,143,353; wherein the active ingredient as described herein is substituted for the active ingredient described therein. The stable solid controlled release composition will have a coating derived from an aqueous dispersion of a hydrophobic acrylic polymer that includes a substrate containing the active ingredient that is overcoated with an aqueous dispersion of a plasticized water-insoluble acrylic polymer. The composition will provide stable dissolution of the active ingredient that is unchanged after exposure to accelerated storage conditions.

The plasticized water-insoluble acrylic polymer contains monomers that can be, for example, an ester of acrylic acid, an ester of methacrylic acid, an alkyl ester of acrylic acid, an alkyl ester of methacrylic acid, and mixtures of any of the foregoing. The compositions can include an additional material that is a polymerizable permeability-enhancing agent, a water-soluble acrylic polymer, a pore-former, and mixtures of any of the foregoing. This will provide controlled release of the active ingredient when the coated substrate is exposed to an environmental fluid.

Additional substances that can be included in the above compositions, as well as methods to make the compositions are described in U.S. Patent No. 6,143,353.

### Example 38

The active ingredient can be prepared as a controlled release composition having microparticles that contain the active ingredient in a polymeric matrix, as described in U.S. Patent No. 5,688,530; wherein the active ingredient as described herein is substituted for the active ingredient described therein.

The polymeric matrix is a biodegradable, biocompatible polymeric matrix of a 40/60 to 60/40 polylactide-co-glycolide ester of a polyol. The polyol is a (C₃₋₆) carbon chain containing alcohol having 3 to 6 hydroxyl groups, or a mono-saccharide and a disaccharide. The esterified polyol will have at least 3 polylactide-co-glycolide chains.

Additional substances that can be included in the above compositions, as well as methods to make the compositions are described in U.S. Patent No. 5,688,530.

### Example 39

The active ingredient can be prepared as a capsule containing a plurality of coated particles that contain a therapeutically effective amount of the active ingredient, as described in U.S. Patent No. 5,656,291; wherein the active ingredient as described herein is substituted for the active ingredient described therein.

The particles are coated with a barrier membrane providing a controlled, preferably pH-independent, release of the active ingredient. The particles will contain at least one water insoluble component (e.g. ethyl cellulose, copolymers of acrylic and methacrylic esters, or natural or synthetic waxes). The water insoluble component will provide a pH-independent drug release.

Additional substances that can be included in the above compositions, as well as methods to make the compositions are described in U.S. Patent No. 5,656,291.

### Example 40

The active ingredient can be prepared as a multilayered controlled release pharmaceutical dosage composition, as described in U.S. Patent No. 5,645,858; wherein the active ingredient as described herein is substituted for the active ingredient described therein.

The multilayered controlled release pharmaceutical dosage composition contains a plurality of coated particles. Each particle contains a core that will contain the active ingredient and a mixture of hydroxypropyl methylcellulose, polyethylene glycol and propylene glycol. The core will be overcoated with a controlled release barrier layer that will contain ethyl cellulose. The controlled release barrier that coats the core will be overcoated with another layer that contains the active ingredient and a mixture of hydroxypropyl methylcellulose, polyethylene glycol and propylene glycol. The second layer that contains the active ingredient will be overcoated with another controlled release barrier layer that will contain ethyl cellulose.

Additional substances that can be included in the above compositions, as well as methods to make the compositions are described in U.S. Patent No. 5,645,858.

### Example 41

The active ingredient can be prepared as a sustained release homogeneous tablet or homogeneous tablet layer, as described in U.S. Patent No. 5,462,747; wherein the active ingredient as described herein is substituted for the active ingredient described therein.

The table or tablet layer can be formed by making a wet granulation using povidone (PVP) in alcohol as the granulating fluid. The wet granulation can then be dried, milled, and blended with a dry powdered erosion promotor, wicking agent, lubricant, and a glidant. The mixture can be compressed to produce a tablet or tablet coating which, upon administration to a patient, results in a long-lasting slow and relatively regular incremental release of the active ingredient. The mixture can be used to produce multilayer tablets for immediate release and sustained release of the active ingredient. An example of a wicking agent is microcrystalline cellulose. An example of an erosion promoter is pregelatinized starch. An example of a lubricant is magnesium stearate. An example of a glidant is silicon dioxide.

Additional substances that can be included in the above compositions, as well as methods to make the compositions are described in U.S. Patent No. 5,462,747.

### Example 42

The active ingredient can be prepared as a sustained release homogeneous tablet or homogeneous tablet layer, as described in U.S. Patent No. 5,393,765; wherein the active ingredient as described herein is substituted for the active ingredient described therein.

The active ingredient of the invention can be prepared as an erodible pharmaceutical composition providing a unique zero order controlled release profile. The erodible composition can contain between about 5% to about 60% w/w of the active ingredient which has a solubility of less than about 80 mg/mL. The composition can also contain about 5% to about 50% w/w of hydroxypropyl methylcellulose having a viscosity from about 50 to about 100 centipoises. The remainder of the composition will consist of inert carriers.

Additional substances that can be included in the above compositions, as well as methods to make the compositions are described in U.S. Patent No. 5,393,765.

### Example 43

The active ingredient can be prepared as a composition for the sustained release of the active ingrediant, as described in U.S. Patent No. 5,356,635; wherein the active ingredient as described herein is substituted for the active ingredient described therein.

The composition includes an amorphous carbohydrate glass matrix containing a suitable carbohydrate and the active ingredient which retards the recrystallization of the carbohydrate and the active ingredient. The matrix will also have a water-insoluble wax dispersed throughout the matrix.

Additional substances that can be included in the above compositions, as well as methods to make the compositions are described in U.S. Patent No. 5,356,635.

### Example 44

The active ingredient can be prepared as a composition for the sustained release of the active ingrediant, as described in U.S. Patent No. 5,328,697; wherein the active ingredient as described herein is substituted for the active ingredient described therein.

The composition will have the active ingredient layered onto non-pareil seeds which are sprayed with a glycine solution. Next, a coating of a wax mixture is applied.

Additional substances that can be included in the above compositions, as well as methods to make the compositions are described in U.S. Patent No. 5,328,697.

### Example 45

The active ingredient can be prepared as a stable sustained release the active ingredient-resin composition for use in liquid carrier for oral administration, as described in U.S. Patent No. 5,186,930; wherein the active ingredient as described herein is substituted for the active ingredient described therein.

The composition contains the active ingredient-resin particle that is coated with a first inner coating of a high temperature melting water-insoluble pharmaceutically acceptable wax and a second outer coating of a pharmaceutically acceptable water-insoluble polymer. The active ingredient-resin particle contains the active ingredient ionically bonded to a pharmaceutically acceptable ion exchange resin particle. The amount of the first inner coating is sufficient to prevent the resin in the active agent-resin particle from swelling and cracking the second outer coating. The active ingredient is released when the complex is placed in a liquid carrier.

Additional substances that can be included in the above compositions, as well as methods to make the compositions are described in U.S. Patent No. 5,186,930.

### Example 46

The active ingredient can be prepared as a stable sustained release the active ingredient-resin composition for use in a liquid carrier for oral administration, as described in U.S. Patent No. 4,892,742; wherein the active ingredient as described herein is substituted for the active ingredient described therein.

The controlled release composition in table form contains a core element that includes about 65-95% by weight of a water soluble the active ingredient, 5-35% by weight of a water insoluble polymeric matrix; and a membrane coating comprising 5-10% by weight of the tablet. The membrane contains a rate-controlling polymer. The insoluble polymeric matrix can contain ethyl cellulose or zein. The insoluble polymer matrix can also contain an oil or wax-like material (e.g. stearic acid, stearyl alcohol, cetyl alcohol, fatty acids, long chain fatty alcohols, carnuba wax, beeswax, white wax, vegetable oil and fatty acid glycerides of C₆₋₁₈ fatty acids). The membrane coating can be cellulose (e.g. ethyl cellulose, mixtures of ethyl cellulose and hydroxypropyl methylcellulose or hydroxypropyl cellulose). The membrane coating can further contain a plasticizer (e.g. triacetin, propylene glycol, polyethylene glycol having a molecular weight of 200 to 800, dibutyl phthalate, dibutyl sebacate, fatty acid, vegetable oils and glycerides of C₆₋₁₈ fatty acids).

Additional substances that can be included in the above compositions, as well as methods to make the compositions are described in U.S. Patent No. 4,892,742.

### Example 47

The active ingredient can be prepared as a stable sustained release dosage composition for use in a liquid carrier for oral administration, as described in U.S. Patent No. 4,781,919; wherein the active ingredient as described herein is substituted for the active ingredient described therein.

The dosage compositions are made of saponified starch-acrylonitrile graft copolymers and the active ingredient. The sustained release injectable dosage forms can contain an effective amount of the active ingredient, and an effective amount of a water insoluble, water swellable, saponified starch acrylonitrile graft copolymer to provide sustained release of the active ingredient upon injection into a patient in need of such treatment.

Additional substances that can be included in the above compositions, as well as methods to make the compositions are described in U.S. Patent No. 4,781,919.

### Example 48

The active ingredient can be prepared as a controlled release dosage composition containing the active ingredient in combination with hydroxypropylmethylcellulose USP 2910, as described in U.S. Patent No. 4,695,591; wherein the active ingredient as described herein is substituted for the active ingredient described therein.

The hydroxypropylmethylcellulose USP 2910 can be less than about one-third of the total dosage form weight of hydroxypropylmethylcellulose USP 2910.

Additional substances that can be included in the above compositions, as well as methods to make the compositions are described in U.S. Patent No. 4,695,591.

### Example 49

The active ingredient can be prepared as a controlled release dosage composition containing an plurality of micronized pellets, as described in U.S. Patent No. 4,529,060; wherein the active ingredient as described herein is substituted for the active ingredient described therein.

The micronized pellets will contain the active ingredient, a water-channelling agent, a wetting agent, and a disintegrant. The mixture can be in the form of a non-compressed pellet having an enteric coat or a sustained release coat permeable to gastrointestinal juices. The micronized pellets can be placed into sustained-release capsules.

Additional substances that can be included in the above compositions, as well as methods to make the compositions are described in U.S. Patent No. 4,524,060.

Additional formulations that can be prepared to include the active ingredient, and methods of preparing the formulations are described, e.g., in U.S. Patent Nos. 6, 419, 953; 6, 251, 432; 6,197,344; 6,150,410; 6,033,685; 6, 010, 718; 5, 705, 190; 5,268,182; 5, 169, 642; 6, 919, 952; 6,395,292; 6,375,978; 6,368,626; 6,342,249; 6,245,357; 6, 174, 597; 6,077,538; 5,650,170; 5, 540, 912; 5. 512, 293; 4,871,548; 4,740,198; 4,692,144; 6,270,799; 5,900,425; 5,707,655; 5,204,121; 5,368,862; 5,366,738; 5,009,895; 4,983,400; 4,919,938; 4,900,755; 4,832,957; 4,639,458; 4,173,626; 5,690,960; 5,660,837; 5,419,918; 4,863,743; 4,634,587; 4,587,118; 4,556,678; 4,508,702; 4,432,965; 4,428,926; 4,428,925; 6,500,454; 6,495,162; 6,492,488; 6,437,000; 6,426,091; 6,419,958; 6,419,953; 6,419,952; 6,416,786; 6,403,120; 6,387,404; 6,372,252; 6,337,091; 6,303,144; 6,284,275; 6,274,171; 6,261,601; 6,254,891; 6,221,395; 6,210,714; 6,197,339; 6,162,466; 6,162,463; 6,156,343; 6,150,410; 6,149,940; 6,136,343; 6,126,967; 6,106,863; 6,099,862; 6,099,859; 6,093,387; 6,090,411; 6,083,533; 6,074,669; 6,056,977; 6,046,177; 6,033,686; 6,033,685; 6,030,642; 6,030,641; 6,027,748; 6,024,982; 5,980,942; 5,945,125; 5,885,615; 5,879,707; 5,874,107; 5,869,100; 5,849,330; 5,846,563; 5,783,212; 5,776,489; 5,736,159; 5,681,583; 5,681,582; 5,667,801; 5,656,291; 5,654,005; 5,645,848; 5,626,874; 5,624,683; 5,614,218; 5,603,956; 5,601,842; 5,593,694; 5,582,837; 5,578,321; 5,576,021; 5,562,915; 5,558,879; 5,554,387; 5,543,155; 5,512,297; 5,508,041; 5,505,962; 5,500,227; 5,498,422; 5,492,700; 5,484,607; 5,466,460; 5,462,747; 5,455,046; 5,433,951; 5,427,799; 5,427,798; 5,407,686; 5,397,574; 5,368,862; 5,362,424; 5,358,723; 5,334,393; 5,334,392; 5,292,534; 5,292,533; 5,283,065; 5,277,912; 5,219,572; 5,200,193; 5,164,193; 5,162,117; 5,126,145; 5,091,189; 5,085,865; 5,075,114; 5,073,380; 5,055,306; 5,051,261; 5,019,398; 5,015,479; 5,007,790; 5,004,613; 5,002,774; 4,983,401; 4,968,509; 4,966,768; 4,933,185; 4,925,676; 4,892,742; 4,882,167; 4,861,590; 4,837,032; 4,824,678; 4,822,619; 4,820,522; 4,816,262; 4,806,359; 4,803,079; 4,803,076; 4,800,083; 4,798,725; 4,795,645; 4,795,642; 4,792,448; 4,784,858; 4,775,535; 4,756,911; 4,734,285; 4,710,384; 4,708,834; 4,695,467; 4,692,337; 4,690,824; 4,666,705; 4,629,620; 4,629,619; 4,610,870; 4,587,118; 4,571,333; 4,557,925; 4,556,678; 4,520,009; 4,505,890; 4,503,031; 4,432,965; 4,415,547; 4,353,887; 4,322,311; 4,308,251; 4,264,573; 4,252,786; 4,173,626; 4,138,475; 4,122,157; 4,002,458; and 3,977,992.

Alternatively, the active ingredient can be administered in a formulation that will form a biodegradable or bioerodible implant, either ex vivo or in vivo. The biodegradable or bioerodible implant, upon degrading in vivo, will release the active ingredient over a suitable period of time. Such formulations that will form a biodegradable implant, either ex vivo or *in vivo*, are described, e.g., in U.S. Patent Nos. RE37,950; 6,461,631; 6,395,293; 6,261,583; 6,180,129; 6,143,314; 6,120,789; 6,113,938; 6,071,530; 5,990,194; 5,945,115; 5,888,533; 5,861,166; 5,780,044; 5,759,563; 5,744,153; 5,739,176; 5,736,152; 5,733,950; 5,702,716; RE35,601; 5,630,808; 5,599,552; 5,487,897; 5,413,572; 5,368,859; 5,340,849; 5,324,519; 5,320,616; 5,278,202; 5,278,201; 5,238,687; 5,234,693; 5,234,692; 5,137,727; 5,112,614; 5,057,318; 4,996,060; 4,455,144; 4,367,741; 4,346,709; 4,340,054; 4,304,232; 4,249,531; 4,142,526; 4,093,709; 4,069,307; and 3,948,254.

## Claims

1. A pharmaceutical composition comprising milnacipran or a pharmaceutically acceptable salt thereof and a pharmaceutically acceptable carrier for use in the therapeutic treatment of a mammal suffering from irritable bowel syndrome (IBS), wherein the pharmaceutical composition does not comprise a neurotransmitter precursor selected from the group consisting of phenylalanine, tyrosine, tryptophan, or a combination thereof.

2. The pharmaceutical composition for use of claim 1, further comprising an antidepressant, an anti-diarrheal, an analgesic, an antispasmodic, an anti-fatigue agent, an anorectic, an antiepileptic drug, a sedative/hypnotic, a laxative, or a combination thereof.

3. The pharmaceutical composition for use of claim 1, further comprising pramipexole.

4. The pharmaceutical composition for use of claim 1, further comprising pregabalin.

5. The pharmaceutical composition for use of claim 1, further comprising neurotonin.

6. The pharmaceutical composition for use of claim 1 wherein the milnacipran is administered up to about 400 mg/day.

7. The pharmaceutical composition for use of claim 1 wherein the milnacipran is administered in about 25 mg/day to about 250 mg/day.

## Patentansprüche

1. Pharmazeutische Zusammensetzung, umfassend Milnacipran oder ein pharmazeutisch verträgliches Salz davon und einen pharmazeutisch verträglichen Träger, zur Verwendung in der therapeutischen Behandlung eines an Reizdarmsyndrom (IBS) leidenden Säugers, wobei die pharmazeutische Zusammensetzung keinen Neurotransmitter-Vorläuferstoff umfasst, der aus der Gruppe ausgewählt ist, bestehend aus Phenylalanin, Tyrosin, Tryptophan oder einer Kombination davon.

2. Pharmazeutische Zusammensetzung zur Verwendung nach Anspruch 1, zusätzlich umfassend ein Antidepressivum, ein Antidiarrhoikum, ein Analgetikum, ein Antispasmodikum, ein Ermüdungsschutzmittel, ein Anorektikum, ein antiepileptisches Medikament, ein Sedativum/Hypnotikum, ein Laxativ oder eine Kombination davon.

3. Pharmazeutische Zusammensetzung zur Verwendung nach Anspruch 1, zusätzlich umfassend Pramipexol.

4. Pharmazeutische Zusammensetzung zur Verwendung nach Anspruch 1, zusätzlich umfassend Pregabalin.

5. Pharmazeutische Zusammensetzung zur Verwendung nach Anspruch 1, zusätzlich umfassend Neurotonin.

6. Pharmazeutische Zusammensetzung nach Anspruch 1, wobei das Milnacipran bis zu ungefähr 400 mg/Tag verabreicht wird.

7. Pharmazeutische Zusammensetzung zur Verwendung nach Anspruch 1, wobei das Milnacipran bei ungefähr 25 mg/Tag bis ungefähr 250 mg/Tag verabreicht wird.

## Revendications

1. Composition pharmaceutique comprenant le milnacipran ou son sel pharmaceutiquement acceptable et un porteur pharmaceutiquement acceptable à utiliser dans le traitement thérapeutique d'un mammifère souffrant du syndrome de l'intestin irritable (SII), la composition pharmaceutique ne comprenant aucun précurseur de neurotransmetteur sélectionné du groupe composé de phénylalanine, tyrosine, tryptophane ou une combinaison de ceux-ci.

2. Composition pharmaceutique pour l'utilisation selon la revendication 1, en outre comprenant un antidépressif, un anti-diarrhéique, un analgésique, un antispasmodique, un antifatigue, un anorexigène, un médicament antiépileptique, un sédatif/hypnotique, un laxatif ou une combinaison de ceux-ci.

3. Composition pharmaceutique pour l'utilisation selon la revendication 1, en outre comprenant le pramipexole.

4. Composition pharmaceutique pour l'utilisation selon la revendication 1, en outre comprenant la pregabaline.

5. Composition pharmaceutique pour l'utilisation selon la revendication 1, en outre comprenant la neurotonine.

6. Composition pharmaceutique selon la revendication 1, dans laquelle le milnacipran est administré jusqu'à environ 400 mg/jour.

7. Composition pharmaceutique pour l'utilisation selon la revendication 1, dans laquelle le milnacipran est administré d'environ 25 mg/jour à 250 mg/jour.
